# EUROPÄISCHE PATENTSCHRIFT

(11) **EP 1 911 436 B1**
(45) Veröffentlichungstag und Bekanntmachung des Hinweises auf die Patenterteilung: **03.07.2013**
(21) Anmeldenummer: 07115070.0
(22) Anmeldetag: 28.08.2007
(51) Int. Cl.: A61K 8/06, A61K 8/02, A61K 8/31, A61K 8/34, A61K 8/37, A61K 8/49, A61K 8/84, A61Q 1/14, A61Q 15/00, A61Q 17/04, A61Q 19/00

(54) **Kaltherstellbare, niedrigviskose und langzeitstabile kosmetische Emulsionen mit kationische Gruppen enthaltenden Coemulgatoren**
Cosmetic emulsions manufactured at low temperature, with low viscosity and long-term stability, with coemulgators containing cationic groups
Emulsions cosmétiques pouvant être produites à froid, peu visqueuses et stables dans le temps, dotées de coémulsifiants contenant des groupes cationiques

(30) Priorität: 06.10.2006 DE 102006047247
(43) Veröffentlichungstag der Anmeldung: 16.04.2008
(73) Patentinhaber: Evonik Goldschmidt GmbH, 45127 Essen (DE)
(72) Erfinder: Meyer, Jürgen, Dr., 48143, Münster (DE); Grüning, Burghard, Dr., 45134, Essen (DE); Hameyer, Peter, 45138, Essen (DE); Leidreiter, Holger, Dr., 45529, Hattingen (DE); Wenk, Hans, Henning Dr., 45138, Essen (DE)

(56) Entgegenhaltungen:
- EP-A- 1 671 615
- EP-A- 1 700 618
- WO-A-02/056841
- DE-A1- 4 405 510
- DE-A1- 10 309 179
- US-A- 4 163 673

## Beschreibung

Gegenstand der Erfindung sind kaltherstellbare langzeitstabile, niedrigviskose, feinteilige Öl-in-Wasser-Emulsionen, deren Herstellung aus vorzugsweise klaren Ölphasen bzw. über vorzugsweise klare bis transparente mikroemulsionsartige Konzentrate, die entsprechenden Ölphasen bzw. mikroemulsionsartigen Konzentrate sowie die Verwendung der erfindungsgemäßen Emulsionen zur Herstellung von kosmetischen, dermatologischen oder pharmazeutischen Zubereitungen sowie zur Herstellung von Reinigungs- und Pflegeemulsionen für Haushalt und Industrie, insbesondere zur Herstellung von Tränkemulsionen für Feuchttücher (Wet Wipes) oder für sprühbare Pflegeemulsionen.

Die erfindungsgemäßen feinteiligen Öl-in-Wasser-Emulsionen sind dabei vorzugsweise frei von polyethylenglykolhaltigen Substanzen ("PEG-frei") und enthalten eine Emulgatormischung, bestehend aus einer primären nichtionischen Emulgatorkomponente, vorzugsweise Polyolpartialestern und einer sekundären kationische Gruppen enthaltenden Emulgatorkomponente, darüber hinaus grenzflächenaktive Cotenside, sowie darüber hinaus Öle, vorzugsweise Ester- und/oder Etheröle und/oder Paraffinöle und gegebenenfalls weitere übliche Hilfs- und Zusatzstoffe.

Emulsionen stellen einen wichtigen Produkttyp im Bereich kosmetischer, dermatologischer und/oder pharmazeutischer Zubereitungen dar. Kosmetische Zubereitungen werden im Wesentlichen zur Hautpflege benutzt. Hautpflege im kosmetischen Sinn ist in erster Linie, dass die natürliche Funktion der Haut als Barriere gegen Umwelteinflüsse (beispielsweise Schmutz, Chemikalien, Mikroorganismen) und gegen den Verlust von körpereigenen Stoffen (beispielsweise Wasser, natürliche Fette, Elektrolyte) gestärkt und/oder wiederhergestellt wird.

Ziel der Hautpflege ist ferner, den durch tägliches Waschen verursachten Fett- und Wasserverlust der Haut auszugleichen und die Weichheit und Glätte der Haut zu erhalten bzw. wieder herzustellen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind schützen und die Hautalterung verzögern.

Pharmazeutische topische Zusammensetzungen enthalten in der Regel ein oder mehrere Medikamente in wirksamer Konzentration. Der Einfachheithalber wird zur eindeutigen Unterscheidung zwischen kosmetischer und medizinischer Anwendung und entsprechenden Produkten auf die gesetzlichen Bestimmungen der Bundesrepublik Deutschland verwiesen (beispielsweise Kosmetikverordnung, Lebensmittel- und Arzneimittelgesetz).

In den letzten Jahren gewannen kosmetische Feuchttücher aufgrund ihrer überaus einfachen und bequemen Verwendbarkeit zunehmende Bedeutung. Zunächst waren nahezu ausschließlich Feuchttücher für Reinigungszwecke auf dem Kosmetikmarkt vertreten, die hauptsächlich wässrige, tensidische Tränklösungen enthielten. Mehr und mehr erschienen aber in der letzten Zeit auch Pflegeprodukte auf dem Markt, die auf Tränkemulsionen basieren und somit zusätzlich eine pflegende Ölkomponente enthalten.

Die meisten dieser kosmetischen Feuchttücher für Körper- und Gesichtspflege sind dabei mit Emulsionen getränkt, die nach der PIT-Emulgiermethode (K. Shinoda, H. Kunieda, Phase properties of emulsions: PIT and HLB, Encycl. of Emulsion Technology, 337-367 (1), 1983 oder Th. Förster, F. Schambil, W. von Rybinski, J. Disp. Sci. And Technology, 13(2), 183-93 (1992)) hergestellt wurden.

Die PIT-Methode macht sich die Tatsache zunutze, dass in einer Öl-in-Wasser(O/W)-Emulsion, die durch nichtionische, Polyethylenglykol enthaltende Emulgatoren ("PEG-haltige Emulgatoren") stabilisiert ist, durch Temperaturerhöhung eine Phaseninversion zu einer Wasser-in-Öl(W/O)-Emulsion induziert werden kann (Phaseninversion; PIT: Phaseninversionstemperatur).

Da in diesem Phaseninversionsbereich die Grenzflächenspannung Wasser-Öl extrem gering ist, können so nach Abkühlung äußerst feinteilige Öl-in-Wasser-Emulsionen erhalten werden. Dazu ist es einerseits notwendig, dass für jedes zu emulgierende System die einzelnen Komponenten der Emulsionen genau aufeinander abgestimmt werden. Das heißt, Emulgatormischungen und Emulgatorkonzentration müssen für unterschiedliche Ölphasen "maßgeschneidert" werden.

Die so hergestellten feinteiligen und niedrigviskosen Emulsionen besitzen eine ausgezeichnete Langzeitstabilität und eignen sich somit gut als Tränklösungen für Feuchttücher. Derartige Systeme sind beispielsweise in EP-B-1 268 740 oder in WO-A-00/04230 beschrieben.

Andererseits ist es bei der PIT-Emulgiertechnik ein grundlegendes Erfordernis, dass die gesamte Emulsion auf Temperaturen oberhalb der Phaseninversionstemperatur erhitzt wird und im Anschluss daran auch wieder abgekühlt werden muss.

In den heutigen Zeiten, in denen Prozessabläufe optimiert und Energiekosten begrenzt werden müssen, bedeutet dies einen deutlichen Nachteil gegenüber Systemen, die diese Aufheiz-/Abkühlkurve nicht durchlaufen müssen.

Aus diesem Grund wären feinteilige, langzeitstabile Emulsionen vorteilhaft, die bei Raumtemperatur hergestellt werden können ("Kaltherstellung") ohne eine zusätzliche Aufheiz-/Abkühlkurve durchlaufen zu müssen.

Nachteilig bei Tränklösungen für Feuchttücher auf Basis von PIT-Emulsionen ist außerdem, dass sie auf der Verwendung PEGhaltiger Emulgatoren beruht. Dies liegt darin begründet, dass in der Regel nur ethoxylierte Emulgatoren eine derart starke Temperaturabhängigkeit ihrer Hydrophilie aufweisen, wie es für temperaturgesteuerte Phaseninversionsvorgänge notwendig ist.

Im Zuge von möglichst natürlichen Kosmetikformulierungen ist ein wichtiges Ziel der Kosmetikforschung, auf Polyethylenglykol ("PEG") enthaltende Emulgatoren verzichten zu können. Daher besteht eine verstärkte Suche nach PEG-freien Alternativlösungen.

Weiterhin ist bekannt, dass ethoxylierte Emulgatoren ein eher wässriges Hautgefühl vermitteln, was durch den Einsatz von beispielsweise Polyglycerinestern sensorisch verbessert werden kann.

So beschreibt WO-A-02/056841 PEG-freie Tränkemulsionen für kosmetische Feuchttücher auf Basis von Polyolpoly-12-hydroxystearaten und Alkylglykosiden. Der Einsatz dieser Emulgatormischungen führt zu einem verbesserten Weichgriff der damit getränkten Papierprodukte und führt auch ansonsten zu verbesserten sensorischen Eigenschaften bei Verwendung der damit hergestellten Feuchttücher. Bei solchen Emulgatorkombinationen ist es allerdings in der Regel schwierig, eine gute Langzeitstabilität der Tränkemulsionen in Kombination mit einer ausreichenden Konservierung zu erreichen.

Insbesondere bei der Herstellung von Feuchttüchern ist eine ausreichende Konservierung der Tränklösungen zwingend erforderlich, um Verkeimungen vorzubeugen. Dabei muss die Konservierung ausreichend sein, um sowohl die Tränklösungen selbst, sowie schließlich auch die getränkten Feuchttücher langzeitig gegenüber Verkeimungen zu schützen.

Als bevorzugte Konservierungsmischungen werden dabei typischerweise Mischungen von Alkylparabenestern und Phenoxyethanol eingesetzt, wie sie etwa unter den Handelsnamen Euxyl® K 300 (Schülke & Mayr) oder Phenonip^{®} (Clariant) kommerziell erhältlich sind.

Die beschriebenen hohen Anforderungen bzgl. einer sicheren Konservierung von Tränklösung und Feuchttüchern machen es dabei notwendig, dass in der Regel relativ große Mengen dieser Alkylparabenester/Phenoxyethanol-Mischungen in den fertigen Tränklösungen eingesetzt werden müssen (0,5 bis 1,0 Ges.-%).

Es ist bekannt, dass der Einsatz dieser Alkylparabenester/Phenoxyethanol-Mischungen emulsionsbelastenden Einfluss hat, da diese Verbindungen sehr grenzflächenaktiv sind und mit Emulgatormolekülen um einen Platz an der Grenzfläche Öl-Wasser konkurrieren. Aufgrund des grenzflächenaktiven Charakters dieser Konservierungsmittel-mischungen kann man sie daher auch als aromatische Cotenside mit konservierenden Eigenschaften beschreiben. Verstärkt wird dieser emulsionsbelastende Effekt im Fall von Tränkemulsionen für Feuchttücher in der Regel durch die erforderlichen hohen Mengen dieser Konservierungsmittel und die geringen Viskositäten der Tränklösungen.

Die EP1700618 beschreibt Öl-in-Wasser-Emulsion, enthaltend eine Emulgatorkombination aus Nichtkohlenhydrat-Polyolpartialestern von linearen oder verzweigten Fettsäuren mit 6 bis 22 C-Atomen (Emulgatorkomponente A) und E mulgatoren auf Kohlenhydratbasis (Emulgatorkomponente B), ein oder mehrere Öle, und mindestens 10 Gew.-% Konservierungsmittel bezogen auf die Gesamtmenge an Emulgatorkomponenten A und B.

Zusammenfassend ist daher festzustellen, dass es mit den im Stand der Technik beschriebenen Emulgatoren oder Emulgatorkombinationen nicht möglich ist, kalt herstellbare, ausreichend konservierte, niedrigviskose, feinteilige und langzeitstabile Emulsionen herzustellen, wie sie typischerweise für Tränkemulsionen oder sprühbare Lotionen verwendet werden.

Eine Aufgabe der vorliegenden Erfindung war es daher, niedrigviskose, feinteilige und langzeitstabile Emulsionen herzustellen, wie sie typischerweise für Tränkemulsionen oder sprühbare Lotionen verwendet werden, d.h., die gleichzeitig
- bei Raumtemperatur hergestellt werden können und
- frei von ethoxylierten Bestandteilen sind und
- die darüber hinaus ohne Problem eine ausreichende Menge konservierend wirkender Verbindungen enthalten können.

Überraschenderweise wurde nun gefunden, dass niedrigviskose, feinteilige und langzeitstabile Öl-in-Wasser-Emulsionen, die hervorragend zum Einsatz als Tränkemulsionen oder in sprühbaren Systemen geeignet sind, bei Raumtemperatur herstellbar sind, wenn eine geeignete Kombination von Emulgatoren auf Basis von Polyolpartialestern und kationische Gruppen enthaltenden Emulgatoren, kosmetischen Ölen und Cotensiden eingesetzt wird.

Diese Emulsionen zeichnen sich neben ihrer einfachen Herstellung und ihrem extrem feinen Dispersionsgrad dadurch aus, dass sie im Wesentlichen frei von ethoxylierten Inhaltsstoffen sind ("PEG-freie" Emulsionssysteme). Die mit Hilfe dieser Tränklösungen hergestellten Feuchttücher zeichnen sich darüber hinaus durch überaus angenehme sensorische Eigenschaften aus. Die eingesetzten kationische Gruppen enthaltenden Emulgatoren können dabei sowohl einen weich machenden Effekt auf den Feuchttüchern verursachen, als auch zu einer besseren Haftung der Tränkemulsion auf Haut und Haar führen.

Mit den erfindungsgemäßen Öl-in-Wasser-Emulsionen sind erstmals kationische Komponenten enthaltende PEG-freie, bei Raumtemperatur einfach herstellbare, niedrigviskose und feinteilige Emulsionen verfügbar, die außerdem durch den erfindungsgemäß bevorzugten Einsatz konservieraktiver aromatischer Cotenside gleichzeitig einfach konserviert werden können und langzeitstabil stabil sind und sich somit insbesondere für den Einsatz als Tränkemulsion für Feuchttücher eignen.

Ein Gegenstand der Erfindung sind daher langzeitstabile, niedrigviskose, feinteilige, im Wesentlichen von ethoxylierten Inhaltsstoffen freie Öl-in-Wasser-Emulsionen mit einem mittleren Radius der Emulsionstropfen von ≥ 20 bis ≤ 500 nm, enthaltend:
A) eine Emulgatormischung bestehend aus:
   a) mindestens einem nichtionischen primären Emulgator ausgewählt aus folgenden Gruppen
      a1) Glycerin- und Polyglycerinpartialester, vorzugsweise hergestellt durch Veresterung von aliphatischen, linearen oder verzweigten, ggfs. ungesättigten und/oder hydroxyfunktionalisierten Carbonsäuren mit einer Kettenlänge von ≥ 6 bist 22 C-Atomen mit Glycerin, Polyglycerinen oder Gemischen aus beiden,
      a2) Sorbitan oder Sorbitolpartialester, vorzugsweise hergestellt durch Veresterung von aliphatischen, linearen oder verzweigten, ggfs. ungesättigten und/oder hydroxyfunktionalisierten Carbonsäuren mit einer Kettenlänge von ≥ 6 bis ≤ 22 C-Atomen mit Sorbitol,
      a3) Kohlenhydratester, bevorzugt Glykosid- oder Sucroseester, vorzugsweise hergestellt durch Veresterung von aliphatischen, linearen oder verzweigten, ggfs. ungesättigten und/oder hydroxyfunktionalisierten Carbonsäuren mit einer Kettenlänge von ≥ 6 bis ≤ 22 C-Atomen mit Mono-, Oligo- oder Polysacchariden,
      a4) (Alkylpoly)-Glykoside, vorzugsweise hergestellt durch Umsetzung von aliphatischen, linearen oder verzweigten, ggfs. ungesättigten und/oder zusätzlich hydroxyfunktionalisierten Alkoholen mit einer Kettenlänge von ≥ 6 bis ≤ 22 C-Atomen mit Mono- oder Polysacchariden,
      oder Mischungen daraus und
   b) mindestens einem kationische Gruppen enthaltenden sekundären Emulgator, enthaltend einen oder mehrere Emulgatoren, die ausgewählt sind aus mindestens einer der Gruppen
      b1) quaternäre Ammoniumverbindungen der allgemeinen Formel I 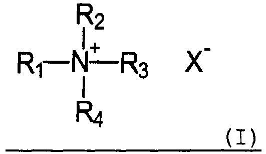 worin
         R₁ ausgewählt ist aus der Gruppe
            - ggfs. verzweigter, ggfs. cyclischer C₆₋₂₂-Alkyl- oder Alkenylrest,
            - R₅-COO-R₆- mit R₅ = ggfs. verzweigter, ggfs. cyclischer, ggfs. hydroxyfunktioneller C₅₋₂₁-Alkyl- oder Alkenylrest und R₆ = ggfs. verzweigter C₂₋₅-Alkylenrest,
            - R₅-CONH-R₆- mit R₅ ggfs. verzweigter, ggfs. cyclischer, ggfs. hydroxyfunktioneller C₅₋₂₁-Alkyl- oder Alkenylrest und R₆ = ggfs. verzweigter C₂₋₅-Alkylenrest,
         R₂, R₃, R₄ unabhängig voneinander ausgewählt aus der Gruppe
            - ggfs. verzweigter, ggfs. cyclischer C₆₋₂₂-Alkyl- oder Alkenylrest,
            - R₅-COO-R₆- mit R₅ = ggfs. verzweigter, ggfs. cyclischer, ggfs. hydroxyfunktioneller C₅₋₂₁-Alkyl- oder Alkenylrest und R₆ = ggfs. verzweigter C₂₋₅-Alkylenrest,
            - R₅-CONH-R₆- mit R₅ = ggfs. verzweigter, ggfs. cyclischer, ggfs. hydroxyfunktioneller C₅₋₂₁-Alkyl- oder Alkenylrest und R₆ = ggfs. verzweigter C₂₋₅-Alkylenrest,
            - C₂₋₄-Alkylrest,
            - C₂₋₄-Alkenylrest,
            - H,
            - 2-Hydroxyethylrest oder 2-Hydroxypropylrest,
         X⁻ ein mit der quaternären Ammoniumverbindung kompatibles Anion wie z. B. Halogenid, Methylsulfat, Ethylsulfat, Glycolat, Lactat, Acetat, Sulfat, Nitrat oder Phosphat,
      b2) Pyridinium-, Oxazolinium-, oder Thiazoliniumverbindungen enthaltend einen oder mehrere Alkylreste mit ≥ 6 bist ≤ 22 C-Atomen,
      b3) Alkylguanidiniumsalze enthaltend Alkylketten mit ≥ 6 bist ≤ 22 C-Atomen,
      b4) Benzylammoniumverbindungen mit einem oder mehreren Alkylresten mit ≥ 6 bis ≤ 22 C--Atomen,
      b5) Aminoxidsalze enthaltend einen oder mehrere Alkylreste mit ≥ 6 bis ≤ 22 C-Atomen,
      b6) Polymere auf Siliconbasis, enthaltend mindestens eine quarternäre Ammoniumgruppe, eine protonierte Aminogruppe oder eine Alkylguanidiniumgruppe,
      b7) Verbindungen der Formel II 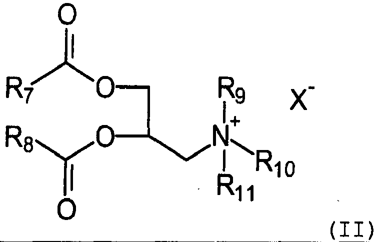 worin
         R₇ und R₈ unabhängig voneinander ggfs. verzweigte, ggfs. cyclische C₆₋₂₂-Alkyl- oder Alkenylreste,
         R₉, R₁₀ und R₁₁ unabhängig voneinander C₁₋₄-Alkylreste sind,
      b8) Verbindungen der Formel III 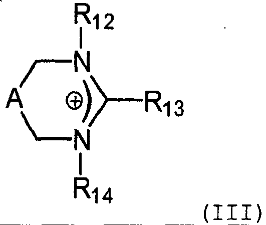 worin
         A direkte Einfach- oder Doppelbindung,
         R₁₂ H oder C₁₋₉-Alkyl,
         R₁₃ H oder C₁₋₂₂-Alkyl,
         R₁₄ ausgewählt aus der Gruppe
            - H,
            - C₁₋₂₂-Alkyl,
            - (CH₂)₂-NHCO-R₁₅ mit R₁₅ = ggfs. verzweigter, ggfs. cyclischer, ggfs. hydroxyfunktioneller C₅₋₂₁-Alkyl- oder Alkenylrest,
            - COR₁₅ mit R₁₅ = ggfs. verzweigter, ggfs. cyclischer, ggfs. hydroxyfunktioneller C₅₋₂₁-Alkyl- oder Alkenylrest, unter der Maßgabe, dass mindestens einer der Reste R₁₂, R₁₃ und R₁₉ mindestens 6 C-Atome enthält,
      b9) Verbindungen der Formel IV 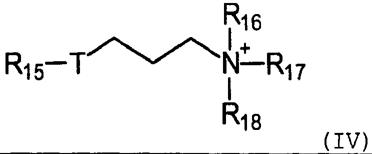 worin
         R₁₅ ggfs. verzweigter C₆₋₂₂ Alkyl- oder Alkenylrest,
         T NH oder O,
         R₁₆, R₁₇, R₁₈ unabhängig voneinander H, C₁₋₄-Alkyl oder 2-Hydroxyalkyl,
         sind,
      b10) Salze von Verbindungen der Formel R₁₉-O-(CH₂)₃-NH-(CH₂)₃-NH₂ mit R₁₉ = ggfs. verzweigter C₆₋₂₂ Alkyl- oder Alkenylrest, bei denen mindestens eine der beiden Amingruppen in protonierter Form vorliegt,
      b11) Verbindungen der allgmeinen Formel V 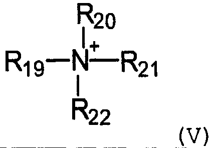 worin
         R₁₉ ausgewählt ist aus
            - ggfs. verzweigter C₆₋₂₂-Alkyl- oder Alkenylrest oder
            - R₂₃-CONH-(CH₂)ₙ- mit R₂₃ = ggfs. verzweigter C₅₋₂₁-Alkyl- oder Alkenylrest und n = 2 bis 4,
         R₂₀ und R₂₁ unabhängig voneinander ausgewählt aus H, C₁₋₄ Alkylrest, C₂₋₄-Alkenylrest oder C₂₋₃-Hydroxyalkylrest und
         R₂₂ ausgewählt aus -CH₂COO⁻, -(CH₂)₂COO⁻, -(CH₂)₃SO₃⁻ und -CH₂CH₂OHCH₂SO₃⁻,
B) ein oder mehrere Cotenside und
C) ein oder mehrere Öle und gegebenenfalls
D) einem oder mehreren polaren Lösungsvermittlern und gegebenfalls
E) übliche Hilfs- und Zusatzstoffe,
mit der Maßgabe, dass der Wasserphasengehalt der Emulsionen ≥ 70 Gew.-% bezogen auf die Gesamtemulsion liegt.

Erfindungsgemäß bevorzugte Emulsionen sind niedrigviskos, feinteilig und langzeitstabil.

Unter niedrigviskos versteht man dabei eine Viskosität, die ein Versprühen der Emulsionen mit üblichen Apparaturen ermöglicht. In der Regel handelt es sich dabei um Emulsionsviskositäten ≤ 4.000 mPas (Brookfield RVT, Spindle 4, 10 rpm (20° C)), vorzugsweise ≤ 2.500 mPas, besonders bevorzugt ≤ 1.000 mPas. Höhere Viskositäten sind einstellbar, jedoch erfindungsgemäß nicht bevorzugt.

Unter feinteilig versteht man dabei einen mittleren Radius der Emulsionstropfen von ≥ 20 bis ≤ 500 nm, bevorzugt ≥ 25 bis ≤ 200 nm und besonders bevorzugt ≥ 30 bis ≤ 120 nm.

Unter langzeitstabil versteht man dabei, dass die erfindungsgemäßen Emulsionen drei Monate bei Raumtemperatur und 1 Monat bei 40° C ohne irreversible Aufrahmung oder sonstigen Anzeichen für Instabilität gelagert werden können.

Die erfindungsgemäßen Öl-in-Wasser-Emulsionen haben üblicherweise einen Wasserphasengehalt von ≥ 70 bis ≤ 99 Gew.-%, vorzugsweise von ≥ 80 bis ≤ 97 Gew.-%.

Zur Wasserphase rechnet man dabei alle Substanzen einer Rezeptur, die aufgrund ihres hydrophilen Charakters dieser Phase beigemischt bzw. in ihr gelöst oder dispergiert werden können. Bezogen auf die erfindungsgemäßen Öl-in-Wasser-Emulsionen gehören somit in jedem Fall Wasser oder etwaige Bestandteile wie Glykole, Polyalkylenglykole, Glycerin, Polyglycerine, Alkohole, wasserlösliche Polymere oder Wirkstoffe zur Wasserphase.

In allen Gegenständen der Erfindung werden die Emulgatormischung (A), die Cotenside (B) und die Öle (C) dabei bevorzugt in Gewichtsanteilen (bezogen auf diese drei Komponenten) von (A) ≥ 10 bis ≤ 30/(B) ≥ 1 bis ≤ 20/C) ≥ 50 bis ≤ 89 und besonders bevorzugt in Gewichtsanteilen von ≥ 20 bis ≤ 25/≥ 3 bis ≤ 15/≥ 60 bis ≤ 77 eingesetzt, wobei sich die Emulgatormischung (A) zusammensetzt aus ≥ 75 bis ≤ 99,9 Gew.-% nichtionischem primären Emulgator (a) und ≥ 0,1 bis ≤ 25 Gew.-% kationische Gruppen enthaltenden sekundärem Emulgator (b).

Üblicherweise basiert die nichtionische primäre Emulgatorkomponente (a) auf Polyglycerinestern, denen vorzugsweise Sorbitanester in einer Menge von ≥ 0 bis ≤ 75 Gew.-%, vorzugsweise ≥ 0 bis ≤ 50 Gew.-%, besonders bevorzugt ≥ 0 bis ≤ 25 Gew.-%, bezogen auf die gesamte primäre Emulgatorkomponente (a), beigemischt werden. Bevorzugt sind dabei Polyglycerin- und Sorbitanpartialester, die als hydrophobe Anteile Fettsäurereste mit einer Kettenlänge von ≥ 10 bis ≤ 18 C-Atomen enthalten. Ganz besonders bevorzugt ist eine Kombination aus Polyglyceryllauraten und Sorbitanlauraten.

Besonders bevorzugt als sekundäre, kationische Gruppen enthaltende Emulgatorkomponenten b) sind dabei Cetrimonium Chloride, Behentrimonium Chloride, Behentrimonium Methosulfate, Dicetyldimonium Chloride, Distearyldimonium Chloride, Palmitamidopropyltrimonium Chloride, Quaternium-18, Ricinolamidopropyl-trimonium Methosulfate, Distearoyl-ethyldimonium Chloride, Distearoylethylhydroxyethylmonium Chloride oder Methosulfate, Dioleoylethylhydroxyethylmonium Chloride oder Methosulfate, Dipalmitoylethyl-hydroxyethylmonium Chloride oder Methosulfate, Cocamido-propylbetain, Cocoamphoacetate sowie kationische polymere Siliconverbindungen wie etwa Quaternium-80 (z.B. ABIL^{®} Quat 3272 (Degussa)) oder Mischungen dieser Verbindungen.

Unter Cotensiden im Sinne der vorliegenden Erfindung werden insbesondere solche Verbindungen verstanden, die sich durch Grenzflächenaktivität auszeichnen, was sich in der Absenkung von Grenzflächenspannungen oder der Einlagerung in Grenzflächenfilme äußern kann, ohne dass diese Substanzen allerdings für sich alleine genommen die für Tenside typische Aggregation zu mizellaren Strukturen in Wasser oder die für Emulgatoren typische Stabilisierung von Emulsionströpfchen zeigen würden.

Unter Cotensiden im Sinne der vorliegenden Erfindung werden außerdem oder alternativ insbesondere solche Verbindungen verstanden, die sich durch einen Octanol-Wasser Partitionskoeffizienten log P oder log K_{ow} auszeichnen, der zwischen 0,8 und 2,2 und bevorzugt zwischen 1 und 2 liegt. Der Octanol-Wasser Verteilungskoeffizient errechnet sich aus dem dekadischen Logarithmus des Quotienten der im Gleichgewicht bei Raumtemperatur gelösten Menge eines Stoffes in Octanol und in Wasser (siehe: O. Fränzle, M. Straskraba in Ullmann's Encyclopedia of Industrial Chemistry, 6th edition, Wiley-VCH, Weinheim, 2000).

Vorteilhafterweise handelt es sich bei den erfindungsgemäßen Cotensiden um nichtionische organische Verbindungen mit 4 bis 14 C-Atomen, die eine oder mehrere polare Gruppen im Molekül enthalten.

Typische bekannte nichtaromatische Cotenside sind aliphatische Alkohole wie Butanol, Pentanol, Hexanol, Heptanol, Octanol, Hexandiol oder Octandiol. Gemäß einer bevorzugten Ausführungsform der Erfindung werden als Cotenside n-Pentanol, n-Hexanol, 1,2-Hexandiol, 1,2-Heptandiol oder 1,2-Octandiol verwendet.

Weiterhin können als Cotenside bevorzugt auch Monoalkylether oder Monoalkylester auf Basis von Glycerin, Ethylenglykol, Propylenglykol oder Diethylenglykol mit Fettsäuren oder Alkoholen mit 6 bis 10 Kohlenstoffatomen verwendet werden.

Gemäß einer bevorzugten Ausführungsform der Erfindung werden als Cotenside aromatische Cotenside verwendet. Unter aromatischen Cotensiden im Sinne der vorliegenden Erfindung werden insbesondere grenzflächenaktive Substanzen verstanden, die eine oder mehrere Arylgruppen enthalten und die für sich alleine genommen in Wasser keine mizellaren Strukturen aufbauen.

Vorteilhafterweise zeichnen sich diese aromatischen Cotenside zusätzlich durch antimikrobielle Eigenschaften aus, d.h. es handelt sich um aromatische Cotenside mit konservierenden Eigenschaften. Die Verwendung solcher Cotenside ermöglicht die Herstellung erfindungsgemäßer O/W-Emulsionen, die idealerweise ohne weitere Konservierungsmittel auskommen. Darüber hinaus ist der Zusatz weiterer üblicher Konservierungsmittel (als Hilfs- und Zusatzstoffe) natürlich möglich, wie sie etwa in DE 102005011785.6 beschrieben sind.

Erfindungsgemäß besonders bevorzugt als aromatische Cotenside mit konservierenden Eigenschaften sind Phenoxyethanol, Phenoxyisopropanol und Benzylalkohol, alleine oder in Kombination mit einem oder mehreren Alkylparabenestern, vorzugsweise Methlyparaben, Ethylparaben, Propylparaben, Isopropylparaben, Butylparaben. Besonders bevorzugt ist die Verwendung von Mischungen von Alkylparabenestern und Phenoxyethanol, wie sie etwa unter den Handelsnamen Euxyl^{®} K 300 (Schülke & Mayr) oder Phenonip^{®} (Clariant) kommerziell erhältlich sind.

Wie erwähnt können auch Mischungen der genannten konservierungsaktiven aromatischen Cotenside mit anderen Cotensiden eingesetzt werden. So kann beispielsweise auch eine Mischung aus Phenoxyethanol und Ethylhexylglycerin verwendet werden, wie sie kommerziell unter dem Namen Euxyl^{®} PE 9010 (Schülke & Mayr) erhältlich ist. Weiterhin können bevorzugt Mischungen aus Phenoxyethanol und 1,2-Octandiol (Capryl Glycol) eingesetzt werden.

Unter Ölen im Sinne der vorliegenden Erfindung werden insbesondere Verbindungen verstanden, ausgewählt aus der Gruppe Guerbetalkohole auf Basis von Fettalkoholen mit 6 bis 20, vorzugsweise 8 bis 10 Kohlenstoffatomen, Ester von linearen C₁₋C₄₄-Fettsäuren mit linearen C₁₋C₂₂-Fettalkoholen, Ester von verzweigten C₁₋C₄₄-Carbonsäuren mit linearen C₁₋C₂₂-Fettalkoholen, Ester von linearen C₁₋C₄₄-Fettsäuren mit verzweigten Alkoholen, Ester von linearen und/oder verzweigten Fettsäuren mit mehrwertigen Alkoholen und/oder Guerbetalkoholen, Triglyceride auf der Basis von C₁₋C₄₄-Fettsäuren, pflanzliche Öle, verzweigte primäre Alkohole, substituierte Cyclohexane, Guerbetcarbonate, Dialk(en)-ylether, Dialk(en)ylcarbonate und/oder aliphatische bzw. naphthenische Kohlenwasserstoffe, Siliconöle, Dimethicone, Cyclomethicone, ethoxylierte und/oder propoxylierte organische Alkohole, ethoxylierte und/oder propoxylierte organische Säuren oder Mischungen daraus. Auch dem Fachmann bekannte Parfumöle können im Sinne der Erfindung als Ölphasen fungieren.

Gemäß einer bevorzugten Ausführungsform der Erfindung werden als Öle Esteröle, Öle auf Etherbasis, Kohlenwasserstoffe, Siliconöle und Mischungen dieser Verbindungen eingesetzt.

Als Esteröle kommen insbesondere Mono- oder Diester von linearen und/oder verzweigten Mono- und/oder Dicarbonsäuren mit ≥ 2 bis ≤ 44 C-Atomen mit linearen und/oder verzweigten (insbesondere 2-Ethylhexanol), gesättigten oder ungesättigten Alkoholen mit ≥ 1 bis ≤ 22 C-Atomen in Betracht. Ebenso sind im erfindungsgemäßen Sinne die Veresterungsprodukte aliphatischer, difunktioneller oder trifunktioneller Alkohole (insbesondere Dimerdiol und/oder Trimerdiol) mit ≥ 2 bis ≤ 36 C-Atomen mit einer oder mehreren monofunktionellen aliphatischen Carbonsäuren mit ≥ 1 bis ≤ 22 C-Atomen geeignet. Weiterhin sind erfindungsgemäß auch Esteröle geeignet, die aromatische Gruppen enthalten.

Die teilweise Verwendung von Esterölen, die bei Raumtemperatur wachsartigen Charakter haben, wie etwa Myristyl Myristate, kann zu einem reichhaltigeren Hautgefühl der Emulsionen führen.

Als Etheröle kommen insbesondere Dialklylether mit ≥ 4 bis ≤ 24 C-Atomen in Betracht. Erfindungsgemäß bevorzugt geeignet sind gesättigte C₆-C₁₈-Dialkylether, wie beispielsweise Di-n-octylether, Di-(2-ethylhexyl)-ether, Laurylmethylether oder Octylbutylether, sowie Didodecylether.

Besonders bevorzugte Ölkomponenten sind die kosmetischen Esteröle Ethylhexylpalmitat, Ethylhexylstearat, Decylcocoat, Diethylhexylcarbonat, Dioctylcarbonat, Cetearylethylhexanoat, Decyloleat, Isocetylpalmitat, Cetearylisononanoat, Hexyllaurat, Isopropylisononaoat, Isopropylstearat, Isopropylpalmitat, Isoproylmyristat, Isopropyllaurat und C₁₂₋₁₅-Alkylbenzoat sowie das kosmetische Etheröl Dicaprylyl Ether und/oder Isohexadecan, Paraffinöl, Octyldodecanol und/oder Cyclopentasiloxane sowie Mischungen der genannten Verbindungen.

Unter "polaren Lösungsvermittlern" im Sinne der vorliegenden Erfindung werden insbesondere polare Verbindungen verstanden, die in Mengen von bis zu 10 Gew.-% den weiter unten beschriebenen Ölphasen beigesetzt werden, um klare Ölphasen zu erhalten. Vorzugsweise handelt es sich dabei um Wasser, Glykole, Polyalkylenglykole, Glycerin, Polyglycerin oder kurzkettige Alkohole wie Ethanol oder iso-Propanol.

Als Hilfs-und Zusatzstoffe können alle dem Fachmann auf diesem Gebiet als Stand der Technik bekannten Hilfs- und Zusatzstoffe wie Öle und Wachse, handelsübliche Tenside oder Emulgatoren, Konsistenzgeber, Verdickungsmittel z.B. auf Polymerbasis, anorganische und organische UV-Lichtschutzfilter, Selbstbräuner, Pigmente, Antioxidantien, Hydrotrope, Deodorant- und Antitranspirantwirkstoffe, Wirkstoffe, Farbstoffe, zusätzliche Konservierungsmittel und Parfüms eingesetzt werden, wie sie etwa in DE 102005011785.6 beschrieben sind. Die Hilfs- und Zusatzstoffe können dabei sowohl der Öl- als auch der Wasserphase bzw. dem Verdünnungswasser im Herstellprozess der Emulsion zugesetzt werden.

Als Wirkstoffe sind dabei insbesondere Tocopherol, Tocopherolacetat, Tocopherolpalmitat, Ascorbinsäure, Desoxyribonucleinsäure, Coenzym Q10, Retinol- und Retinylderivate, Bisabolol, Allantoin, Phytantriol, Panthenol, AHA-Säuren, Aminosäuren, Hyaluronsäure, Creatin (und Creatinderivate), Creatinin, Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Salicylsäure, Zinkricinoleat, Pflanzenextrakte und Vitaminkomplexe bevorzugt.

Die erfindungsgemäßen O/W-Emulsionen sind prinzipiell unter Benutzung eines einfachen Rührwerkzeugs herstellbar. Dabei wird kein zusätzlicher Homogenisierschritt benötigt.

Die Herstellung erfolgt bevorzugt bei Raumtemperatur durch direktes Eingießen einer klaren, einphasigen Ölphase, enthaltend eine Emulgatormischung, Cotenside, Öle und ggfs. übliche Hilfs- und Zusatzstoffe, ins Verdünnungswasser. In vielen Fällen kann die Herstellung auch umgekehrt durch Zugabe des Verdünnungswassers zur vorgelegten klaren Ölphase erfolgen. Falls nötig, kann die Ölphase durch Zusatz von bis zu 10 Gew.-% eines polaren Lösungsvermittlers in eine klare Phase verwandelt werden. Solche polaren Lösungsvermittler können sein Wasser, Glykole, Polyalkylenglykole, Glycerin, Polyglycerine oder kurzkettige Alkohole wie Ethanol oder iso-Propanol. Bevorzugt wird als polarer Lösungsvermittler Wasser verwendet.

Homogene, klare Ölphasen sind zur Herstellung erfindungsgemäßer, feinteiliger O/W-Emulsionen vorteilhaft. Die Verwendung trüber Ölphasen führt in der Regel zu grobteiligeren Emulsionen, deren Langzeitstabilität oft unzureichend ist. Der Übergang von klaren zu trüben Ölphasen ist dabei fließend. Der Trübungsgrad, bei dem noch Emulsionen mit ausreichender Langzeitstabilität herstellbar sind, ist abhängig von Art und Menge der eingesetzten Komponenten und in diesen Grenzfällen individuell zu bestimmen.

Alternativ zur genannten Methode können erfindungsgemäße feinteilige Öl-in-Wasser-Emulsionen auch über die Zwischenstufe eines klaren bis transparenten mikroemulsionsartigen Konzentrats erfolgen. Dieses Konzentrat besteht in der Regel aus > 30 bis ≤ 90 Gew.-% Ölphase, bevorzugt aus ≥ 40 bis ≤ 80 Gew.-% Ölphase, enthaltend eine Emulgatormischung, Cotenside, Öle und ggfs. polare Lösungsvermittler und/oder übliche Hilfs- und Zusatzstoffe.

Diese klaren bis transparenten mikroemulsionsartigen Konzentrate werden vorzugsweise bei Raumtemperatur durch Einrühren von Wasser in die Ölphase hergestellt. Zur Herstellung dieser Konzentrate können auch trübe Ölphasen verwendet werden. Dabei ist der optimale Wassergehalt der Konzentrate rezepturabhängig (z.B. vom eingesetzten Öl), liegt aber in der Regel bei ≥ 10 bis < 70 Gew.-%, bevorzugt bei ≥ 20 bis ≤ 60 Gew.-%.

Diese mikroemulsionsartigen Konzentrate können schließlich zu erfindungsgemäßen Öl-in-Wasser-Emulsionen verdünnt werden. Dabei kann sowohl die Herstellung der mikroemulsionsartigen Konzentrate als auch der abschließende Verdünnungsschritt bei Raumtemperatur unter Verwendung eines einfachen Rührwerkzeugs erfolgen.

Ein weiterer Gegenstand der Erfindung sind daher Ölphasen enthaltend:
A) eine Emulgatormischung bestehend aus:
   a) mindestens einem nichtionischen primären Emulgator wie oben definiert und
   b) mindestens einem eine kationische Gruppen enthaltenden sekundären Emulgator wie oben definiert
B) ein oder mehrere Cotenside und
C) ein oder mehrere Öle und
D) ≥ 0 bis < 10 Gew.-% (bezogen auf die gesamte Ölphase) eines oder mehrerer polarer Lösungsvermittler und gegebenfalls
E) übliche Hilfs- und Zusatzstoffe.

Erfindungsgemäß bevorzugt sind dabei homogene und klare Ölphasen.

Diese Ölphasen sind herstellbar nach den bekannten Verfahren des Standes der Technik. Beispielsweise können die Ölphasen je nach Konsistenz und Konzentration der verwendeten Komponenten bei Temperaturen im Bereich von ≥ 20 bis ≤ 75 °C durch einfaches Vermischen der Komponenten hergestellt werden. Diese Ölphasen sind bei Raumtemperatur zur Herstellung der erfindungsgemäßen Öl-in-Wasser-Emulsionen verwendbar.

Ein weiterer Gegenstand der Erfindung ist ein Verfahren zur Herstellung erfindungsgemäßer Öl-in-Wasser-Emulsionen, welches dadurch gekennzeichnet ist, dass diese klaren Ölphasen vorzugsweise bei Temperaturen < 40 °C, insbesondere Raumtemperatur, mit einer entsprechenden Wasserphase unter an sich bekannten Bedingungen auf einen Gesamtwasserphasengehalt von ≥ 70 Gew.-%, bevorzugt ≥ 80 Gew.-%, eingestellt wird.

Ein weiterer Gegenstand der Erfindung ist ein Verfahren zur Herstellung erfindungsgemäßer Öl-in-Wasser-Emulsionen, welches dadurch gekennzeichnet ist, dass klare bis transparente mikroemulsionsartige Konzentrate enthaltend:
A) eine Emulgatormischung bestehend aus:
   a) mindestens einem nichtionischen primären Emulgator wie oben definiert und
   b) mindestens einem eine kationische Gruppen enthaltenden sekundären Emulgator wie oben definiert,
B) ein oder mehrere Cotenside und
C) ein oder mehrere Öle und gegebenfalls
D) einem oder mehreren polaren Lösungsvermittlern und gegebenfalls,
E) übliche Hilfs- und Zusatzstoffe,
mit der Maßgabe, dass der Gesamtwasserphasengehalt der mikroemulsionsartigen Konzentrate ≥ 10 bis < 70 Gew.-%, bezogen auf das gesamte Konzentrat, beträgt bei Raumtemperatur mit einer entsprechenden Wasserphase unter an sich bekannten Bedingungen auf einen Gesamtwasserphasengehalt von ≥ 70 Gew.-%, bevorzugt ≥ 80 Gew.-%, eingestellt wird.

Ein weiterer Gegenstand der Erfindung ist die Verwendung der erfindungsgemäßen Emulsionen zur Herstellung von kosmetischen, dermatologischen oder pharmazeutischen Zubereitungen. Insbesondere steht dabei der Einsatz als Tränklösungen zur Herstellung von Feuchttüchern, ganz besonders von kosmetischen Feuchttüchern zur Pflege und Reinigung der Haut und Hautanhangsgebilden im Vordergrund.

Ein weiterer Gegenstand der Erfindung ist die Verwendung der Emulsionen in kosmetischen Reinigungs- und Pflegezubereitungen für Haut- und Hautanhangsgebilde. Insbesondere steht dabei die Verwendung in sprühbaren Zubereitungen im Vordergrund, wie sie etwa für Gesichts- und Körperpflegeprodukte, Babypflege, Sonnenschutzpräparate, Make-up Remover sowie Antiperspirantien/Deodorantien sowie zur Haarpflege verwendet werden.

Die erfindungsgemäßen Öl-in-Wasser-Emulsionen eignen sich auch hervorragend sowohl zur Herstellung von Reinigungs- und Pflegefeuchtüchern als auch zur direkten Anwendung in Form von sprühbaren Emulsionssystemen zur Reinigung und Pflege von Oberflächen in Haushalt und Industrie, wie etwa der Textilpflege, der Lederpflege, der Pflege und Reinigung von metallischen oder nichtmetallischen Oberflächen, beispielsweise zur Reinigung und Pflege von Automobilen oder Möbeln.

Ein weiterer Gegenstand der Erfindung ist demnach die Verwendung der Emulsionen zur Herstellung von Reinigungs- und Pflegemitteln für Haushalt und Industrie, wie Textilien, Leder, Kunststoffe, metallische und nichtmetallische Oberflächen. Insbesondere steht dabei der Einsatz als Tränklösungen zur Herstellung von Feuchttüchern sowie die Verwendung in sprühbaren Zubereitungen im Vordergrund.

Die hier beschriebene technische Lehre ermöglicht auf einfache Weise bei Raumtemperatur, PEG-freie, niedrigviskose, feinteilige und langzeitstabile Öl-in-Wasser-Emulsionen herzustellen, die bereits eine ausreichende Konservierung aufweisen.

Die dabei eingesetzten sekundären, kationische Gruppen enthaltenden Emulgatoren können dabei sowohl einen weichmachenden Effekt auf den Feuchttüchern verursachen, als auch zu einer besseren Haftung der Tränkemulsion auf Haut und Haar führen, sowie die sensorischen Eigenschaften der Emulsionen generell vorteilhaft beeinflussen.

### Ausführungsbeispiele:

Die folgenden Beispiele sollen den Gegenstand der Erfindung näher erläutern, ohne ihn auf diese Beispiele einzuschränken. Die Konzentrationsangaben in allen Beispielen sind als Gew.-% angegeben.

Die Herstellung der erfindungsgemäßen Ölphasen, der erfindungsgemäßen mikroemulsionsartigen Konzentrate und der erfindungsgemäßen Emulsionen erfolgte unter Verwendung eines einfachen manuellen Rührwerkzeugs. Nach Herstellung der entsprechenden Ölphasen erfolgte sowohl die Überführung in erfindungsgemäße mikroemulsionsartige Konzentrate und in erfindungsgemäße Öl-in-Wasser-Emulsionen bei Temperaturen < 30° C.

### Beispielemulgatoren 1 bis 9:

Beschreibung der in den Beispielformulierungen eingesetzten Emulgatorsysteme (die Gesamtprozentzahl pro Emulgatorsystem addiert sich jeweils auf 100):

| Emulgator 1: | |
|---|---|
| Emulgatorkomponente A: | 99,1 % Polyglyceryl-4 Laurate¹⁾ |
| Emulgatorkomponente B: | 0,9 % Cetyltrimonium Chloride²⁾ |

| Emulgator 2: | |
|---|---|
| Emulgatorkomponente A: | 98,0 % Polyglyceryl-4 Laurate¹⁾ |
| Emulgatorkomponente B: | 2,0 % Quaternium-80³⁾ |

| Emulgator 3: | |
|---|---|
| Emulgatorkomponente A: | 79,4 % Polyglyceryl-4 Laurate¹⁾ |
| | 19,8 % Sorbitan Laurate⁴⁾ |
| Emulgatorkomponente B: | 0,8 % Cetyltrimonium Chloride²⁾ |

| Emulgator 4: | |
|---|---|
| Emulgatorkomponente A: | 79,2 % Polyglyceryl-4 Laurate¹⁾ |
| | 19,8 % Sorbitan Laurate⁴⁾ |
| Emulgatorkomponente B: | 1,0 % Cetyltrimonium Chloride²⁾ |

| Emulgator 5: | |
|---|---|
| Emulgatorkomponente A: | 99,4 % Polyglyceryl-4 Laurate¹⁾ |
| Emulgatorkomponente B: | 0,6 % Cetyltrimonium Chloride²⁾ |

| Emulgator 6: | |
|---|---|
| Emulgatorkomponente A: | 96,6 % Polyglyceryl-4 Laurate¹⁾ |
| Emulgatorkomponente B: | 3,4 % Quaternium-80³⁾ |

| Emulgator 7: | |
|---|---|
| Emulgatorkomponente A: | 98,7 % Polyglyceryl-4 Laurate¹⁾ |
| Emulgatorkomponente B: | 1,3 % Palmitamidopropyltrimonium |
| | Chloride⁵⁾ |

| Emulgator 8: | |
|---|---|
| Emulgatorkomponente A: | 68,9 % Polyglyceryl-4 Laurate¹⁾ |
| | 29, 5 % Sorbitan Laurate⁴⁾ |
| Emulgatorkomponente B: | 1,6 % Cetyltrimonium Chloride²⁾ |

| Emulgator 9: | |
|---|---|
| Emulgatorkomponente A: | 99,3 % Polyglyceryl-4 Laurate¹⁾ |
| Emulgatorkomponente B: | 0,7 % Dicetyldimonium Chloride⁶⁾ |

| | |
|---|---|
| 1) TEGO^{®} Care PL 4 (Goldschmidt GmbH) 2) VARISOFT^{®} 300 (Degussa) 3) ABIL^{®} Quat 3272 (Degussa) 4) TEGO^{®} SML (Degussa) 5) VARISOFT^{®} PATC (Degussa) 6) VARISOFT^{®} 432 PPG (Degussa) | |

### Beispiele für klare Ölphasen 1 bis 5:

Diese Beispiele sollen insbesondere zeigen, wie sich erfindungsgemäße klare Ölphasen zusammensetzen können, die in einem weiteren Schritt bei Raumtemperatur zu erfindungsgemäßen, feinteiligen Öl-in-Wasser-Emulsionen verarbeitet werden können (siehe Beispielemulsionen 1 bis 5).

| Klare Ölphasen | 1 in % | 2 in % | 3 in % | 4 in % | 5 in % |
|---|---|---|---|---|---|
| Emulgator 1 | 21,7 | | | | |
| Emulgator 2 | | 21,4 | | | |
| Emulgator 3 | | | 21,7 | | |
| Emulgator 4 | | | | 21,7 | |
| Emulgator 5 | | | | | 21,8 |
| Isopropyl Palmitate | | | | | 64,9 |
| Ethylhexyl Palmitate | | | | 64,4 | |
| Diethylhexyl Carbonate | 64,6 | 63,0 | | | |
| Decyl Cocoate | | | 64,7 | | |
| Phenoxyethanol | 12,0 | 11,8 | 12,1 | 12,0 | 12,1 |
| Wasser | 1,7 | 3,8 | 1,5 | 1,9 | 1,2 |

### Beispiele für klare bis transparente mikroemulsionsartige Konzentrate 1 bis 5:

Diese Beispiele sollen insbesondere zeigen, wie sich erfindungsgemäße klare bis transparente mikroemulsionsartige Konzentrate zusammensetzen können, die in einem weiteren Schritt bei Raumtemperatur zu erfindungsgemäßen, feinteiligen Öl-in-Wasser-Emulsionen verarbeitet werden können (siehe Beispielemulsionen 6 bis 10).

| Mikroemulsionsähnliche Konzentrate | 1 in % | 2 in % | 3 in % | 4 in % | 5 in % |
|---|---|---|---|---|---|
| Emulgator 1 | 11,0 | | | | |
| Emulgator 6 | | 10,9 | | | |
| Emulgator 7 | | | 11,0 | | |
| Emulgator 8 | | | | 10,9 | |
| Emulgator 9 | | | | | 12,0 |
| Cetearyl Isononanoate | | | | 32,3 | |
| Isopropyl Palmitate | | | | | 17,9 |
| Paraffinöl (25 mPas bei 30° C) | | | | | 17,9 |
| Diethylhexyl Carbonate | 32,6 | 31,7 | 32,4 | | |
| Phenoxyethanol | 6,1 | 5,9 | 6,1 | 6,0 | 1,9 |
| Capryl Gylcol⁷⁾ | | | | | 1,9 |
| Wasser | 50,3 | 51,5 | 50,5 | 50,8 | 48,4 |

| | | | | | |
|---|---|---|---|---|---|
| ⁷⁾Dermosoft^{®} Octiol (Dr. Straetmans) (1,2-Octandiol) | | | | | |

### Beispielemulsionen 1 bis 10:

Die Emulsionen 1 bis 10 sollen den Aufbau erfindungsgemäßer Emulsionen beispielhaft veranschaulichen.

Dabei wurden die Emulsionen 1 bis 5 durch Eingießen der klaren Ölphasen (Beispiele 1 bis 5 (s.o.)) in Wasser bei Raumtemperatur unter Verwendung eines einfachen manuellen Rührwerkzeugs hergestellt.

Die Emulsionen 6 bis 10 wurden ebenfalls bei Raumtemperatur durch Verdünnen der klaren bis transparenten mikroemulsionsartigen Konzentrate (entsprechende Beispiele 1 bis 5 (s.o.)) unter Verwendung eines einfachen manuellen Rührwerkzeugs hergestellt.

Dabei sind die Beispielemulsionen 1 und 6 in ihrer Zusammensetzung identisch. Lediglich die Herstellung der Emulsionen erfolgte auf unterschiedlichen Wegen (1: direkt aus Verdünnung der Ölphase; 6: über das mikroemulsionsähnliche Konzentrat). Viskosität, Blauschimmer und Stabilität der beiden Emulsionsbeispiele sind absolut vergleichbar.

Alle Beispielemulsionen sind niedrigviskos, feinteilig und langzeitstabil.

| Beispielemulsionen | 1 in % | 2 in % | 3 in % | 4 in % | 5 in % |
|---|---|---|---|---|---|
| Emulgator 1 | 1,3 | | | | |
| Emulgator 2 | | 1,3 | | | |
| Emulgator 3 | | | 1,3 | | |
| Emulgator 4 | | | | 1,3 | |
| Emulgator 5 | | | | | 1,3 |
| Isopropyl Palmitate | | | | | 3,7 |
| Ethylhexyl Palmitate | | | | 3,7 | |
| Diethylhexyl Carbonate | 3,7 | 3,7 | | | |
| Decyl Cocoate | | | 3,7 | | |
| Phenoxyethanol | 0,7 | 0,7 | 0,7 | 0,7 | 0,7 |
| Wasser | 94,3 | 94,3 | 94,3 | 94,3 | 94,3 |

| Beispielemulsionen | 6 in % | 7 in % | 8 in % | 9 in % | 10 in % |
|---|---|---|---|---|---|
| Emulgator 1 | 1,3 | | | | |
| Emulgator 6 | | 1,3 | | | |
| Emulgator 7 | | | 1,3 | | |
| Emulgator 8 | | | | 1,3 | |
| Emulgator 9 | | | | | 5,0 |
| Cetearyl Isononanoate | | | | 3,7 | |
| Isopropyl Palmitate | | | | | 7,5 |
| Paraffinöl (25 mPas bei 30° C) | | | | | 7,5 |
| Diethylhexyl Carbonate | 3,7 | 3,7 | 3,7 | | |
| Phenoxyethanol | 0,7 | 0,7 | 0,7 | 0,7 | 0,8 |
| Capryl Gylcol⁷⁾ | | | | | 0,8 |
| Wasser | 94,3 | 94,3 | 94,3 | 94,3 | 78,4 |

### Partikelgrößenbestimmung durch Dynamische Lichtstreuung:

Beispielhaft für den extrem feinen Dispersionsgrad der erfindungsgemäßen Emulsionen wurde mit Hilfe von Dynamischer Lichtstreuung die Partikelgröße einzelner Beispielemulsionen charakterisiert.

Dynamische Lichtstreuung beruht auf der Analyse der Schwankungen der Streulichtintensität diffundierender Partikel in Lösung. Für verdünnte Lösungen kann dadurch der Diffusionskoeffizient der Teilchen in Lösung bestimmt werden, der über die Stokes-Einstein-Gleichung in einen mittleren hydrodynamischen Radius <rₕ> der Teilchen (hier: Emulsionströpfchen) umgerechnet werden kann.

Im vorliegenden Fall wurden die genannten Beispielemulsionen mit vollentsalztem Wasser auf einen Ölphasengehalt von jeweils 0,5 % verdünnt und mit Hilfe eines Dynamischen Lichtstreugeräts der Firma Malvern Instruments (HPPS 3.1) bei 25° C charakterisiert. Angegeben sind jeweils die intensitätsgewichteten Mittelwerte aus jeweils drei Messungen mit jeweils 100 Sekunden Messzeit.

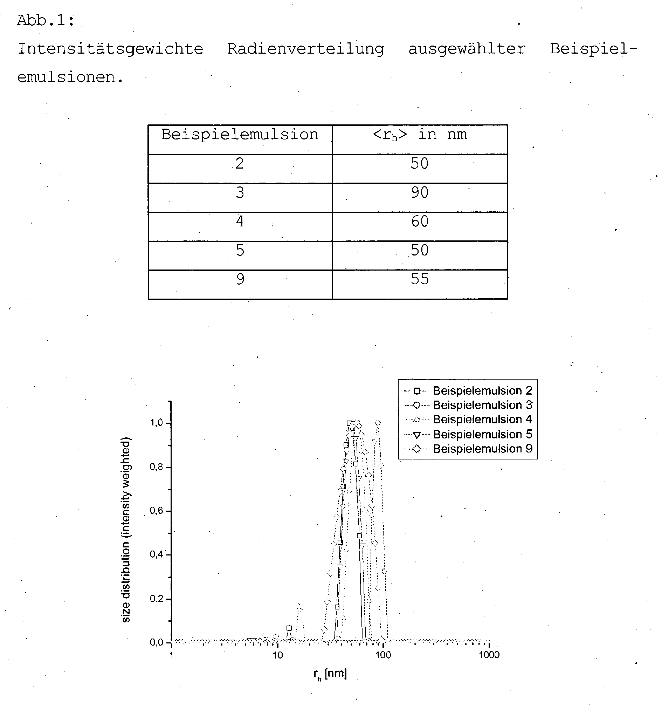

Die vollständigen intensitätsgewichteten Radienverteilungen der ausgewählten Beispielemulsionen sind in Abb. 1 dargestellt.

## Patentansprüche

1. Langzeitstabile, niedrigviskose, feinteilige, im Wesentlichen von ethoxylierten Inhaltsstoffen freie Öl-in-Wasser-Emulsionen mit einem mittleren Radius der Emulsionstropfen von ≥ 20 bis ≤ 500 nm, enthaltend
A) eine Emulgatormischung bestehend aus
a) mindestens einem nichtionischen primären Emulgator ausgewählt aus folgenden Gruppen
a1) Glycerin- und Polyglycerinpartialester, vorzugsweise hergestellt durch Veresterung von aliphatischen, linearen oder verzweigten, ggfs. ungesättigten und/oder hydroxyfunktionalisierten Carbonsäuren mit einer Kettenlänge von ≥ 6 bis ≤ 22 C-Atomen mit Glycerin, Polyglycerinen oder Gemischen aus beiden,
a2) Sorbitan oder Sorbitolpartialester, vorzugsweise hergestellt durch Veresterung von aliphatischen, linearen oder verzweigten, ggfs. ungesättigten und/oder hydroxyfunktionalisierten Carbonsäuren mit einer Kettenlänge von ≥ 6 bis ≤ 22 C-Atomen mit Sorbitol,
a3) Kohlenhydratester, bevorzugt Glykosid- oder Sucroseester, vorzugsweise hergestellt durch Veresterung von aliphatischen, linearen oder verzweigten, ggfs. ungesättigten und/oder hydroxyfunktionalisierten Carbonsäuren mit einer Kettenlänge von ≥ 6 bis ≤ 22 C-Atomen mit Mono-, Oligo- oder Polysacchariden,
a4) (Alkylpoly)-Glykoside, vorzugsweise hergestellt durch Umsetzung von aliphatischen, linearen oder verzweigten, ggfs. ungesättigten und/oder zusätzlich hydroxyfunktionalisierten Alkoholen mit einer Kettenlänge von ≥ 6 bis ≤ 22 C-Atomen mit Mono- oder Polysacchariden,
oder Mischungen daraus und
b) mindestens einem kationische Gruppen enthaltenden sekundären Emulgator, enthaltend einen oder mehrere Emulgatoren, die ausgewählt sind aus mindestens einer der Gruppen
b1) quaternäre Ammoniumverbindungen der allgemeinen Formel I 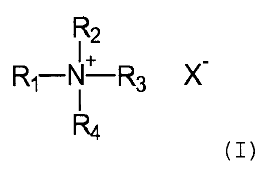 worin
R₁ ausgewählt ist aus der Gruppe
- ggfs. verzweigter, ggfs. cyclischer C₆₋₂₂-Alkyl- oder Alkenylrest,
- R₅-COO-R₆- mit R₅ = ggfs. verzweigter, ggfs. cyclischer, ggfs. hydroxyfunktioneller C₅₋₂₁-Alkyl- oder Alkenylrest und R₆ = ggfs. verzweigter C₂₋₅-Alkylenrest,
- R₅-CONH-R₆- mit R₅ = ggfs. verzweigter, ggfs. cyclischer, ggfs. hydroxyfunktioneller C₅₋₂₁-Alkyl- oder Alkenylrest und R₆ = ggfs. verzweigter C₂₋₅-Alkylenrest,
R₂, R₃, R₄ unabhängig voneinander ausgewählt aus der Gruppe
- ggfs. verzweigter, ggfs. cyclischer C₆₋₂₂-Alkyl- oder Alkenylrest,
- R₅-COO-R₆- mit R₅ = ggfs. verzweigter, ggfs. cyclischer, ggfs. hydroxyfunktioneller C₅₋₂₁-Alkyl- oder Alkenylrest und R₆ = ggfs. verzweigter C₂₋₅-Alkylenrest,
- R₅-CONH-R₆- mit R₅ = ggfs. verzweigter, ggfs. cyclischer, ggfs. hydroxyfunktioneller C₅₋₂₁-Alkyl- oder Alkenylrest und R₆ = ggfs. verzweigter C₂₋₅-Alkylenrest,
- C₁₋₄-Alkylrest,
- C₂₋₄-Alkenylrest,
- H,
- 2-Hydroxyethylrest oder 2-Hydroxypropylrest,
X⁻ ein mit der quaternären Ammoniumverbindung kompatibles Anion wie z. B. Halogenid, Methylsulfat, Ethylsulfat, Glycolat, Lactat, Acetat, Sulfat, Nitrat oder Phosphat,
b2) Pyridinium-, Oxazolinium-, oder Thiazoliniumverbindungen enthaltend einen oder mehrere Alkylreste mit ≥ 6 bis ≤ 22 C-Atomen,
b3) Alkylguanidiniumsalze enthaltend Alkylketten mit ≥ 6 bis ≤ 22 C-Atomen,
b4) Benzylammoniumverbindungen mit einem oder mehreren Alkylresten mit ≥ 6 bis ≤ 22 C-Atomen,
b5) Aminoxidsalze enthaltend einen oder mehrere Alkylreste mit ≥ 6 bis ≤ 22 C-Atomen,
b6) Polymere auf Siliconbasis, enthaltend mindestens eine quarternäre Ammoniumgruppe, eine protonierte Aminogruppe oder eine Alkylguanidiniumgruppe,
b7) Verbindungen der Formel II 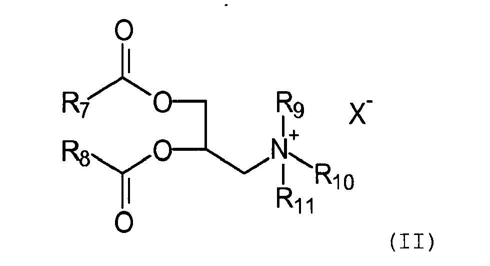 worin
R₇ und R₈ unabhängig voneinander ggfs. verzweigte, ggfs. cyclische C₆₋₂₂-Alkyl- oder Alkenylreste,
R₉, R₁₀ und R₁₁ unabhängig voneinander C₁₋₄-Alkylreste sind,
b8) Verbindungen der Formel III 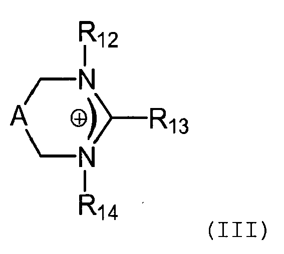 worin
A direkte Einfach- oder Doppelbindung,
R₁₂ H oder C₁₋₄-Alkyl,
R₁₃ H oder C₁₋₂₂-Alkyl,
R₁₄ ausgewählt aus der Gruppe
- H,
- C₁₋₂₂-Alkyl,
- (CH₂)₂-NHCO-R₁₅ mit R15 = ggfs. verzweigter, ggfs. cyclischer, ggfs. hydroxyfunktioneller C₅₋₂₁-Alkyl- oder Alkenylrest,
- COR₁₅ mit R₁₅ = ggfs. verzweigter, ggfs. cyclischer, ggfs. hydroxyfunktioneller C₅₋₂₁-Alkyl- oder Alkenylrest,
unter der Maßgabe, dass mindestens einer der Reste R₁₂, R₁₃ und R₁₄ mindestens 6 C-Atome enthält,
b9) Verbindungen der Formel IV 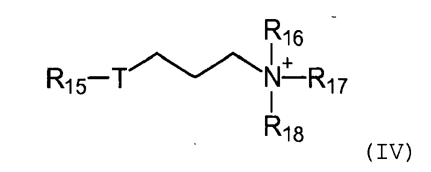 worin
R₁₅ ggfs. verzweigter C₆₋₂₂ Alkyl- oder Alkenylrest,
T NH oder O,
R₁₆, R₁₇, R₁₈ unabhängig voneinander H, C₁₋₄-Alkyl oder 2-Hydroxyalkyl,
sind,
b10) Salze von Verbindungen der Formel R₁₉-O-(CH₂)₃-NH-(CH₂)3-NH₂ mit R₁₉ = ggfs. verzweigter C₆₋₂₂ Alkyl- oder Alkenylrest, bei denen mindestens eine der beiden Amingruppen in protonierter Form vorliegt,
b11)Verbindungen der allgmeinen Formel V 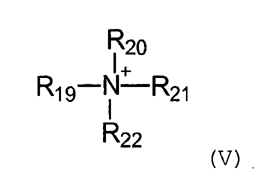 worin
R₁₉ ausgewählt ist aus
- ggfs. verzweigter C₆₋₂₂-Alkyl- oder Alkenylrest oder
- R₂₃-CONH- (CH₂) n- mit R₂₃ = ggfs. verzweigter C₅₋₂₁-Alkyl- oder Alkenylrest und n = 2 bis 4,
R₂₀ und R₂₁ unabhängig voneinander ausgewählt aus H, C₁₋₄ Alkylrest, C₂₋₄-Alkenylrest oder C₂₋₃-Hydroxyalkylrest und
R₂₂ ausgewählt aus -CH₂COO⁻, -(CH₂)₂COO, -(CH₂)₃SO₃⁻ und -CH₂CH₂OHCH₂SO₃⁻,
B) ein oder mehrere Cotenside und
C) ein oder mehrere Öle und gegebenenfalls,
D) einem oder mehreren polaren Lösungsvermittlern und gegebenfalls
E) übliche Hilfs- und Zusatzstoffe,
mit der Maßgabe, dass der Wasserphasengehalt der Emulsionen ≥ 70 Gew.-%, bezogen auf die Gesamtemulsion, liegt.

2. Eine Öl-in-Wasser-Emulsion nach Anspruch 1, **dadurch gekennzeichnet, dass** sie einen Wasserphasengehalt von ≥ 80 bis ≤ 99 Gew.-% aufweist.

3. Eine Öl-in-Wasser-Emulsion nach Anspruch 1 oder 2, **dadurch gekennzeichnet, dass** Emulgatormischung (A), Cotenside (B) und Öle (C) in Gewichtsanteilen von ≥ 10 bis ≤ 30 (A), ≥ 1 bis ≤ 20 (B) und ≥ 50 bis ≤ 89 (C) bezogen auf die Summe dieser Komponenten vorliegen.

4. Eine Öl-in-Wasser-Emulsion nach einem oder mehreren der Ansprüche 1 bis 3, **dadurch gekennzeichnet, dass** sich die Emulgatormischung (A) zusammensetzt aus ≥ 75 bis ≤ 99,9 Gew.-% nichtionischer primärer Emulgatorkomponente (a) und ≥ 0,1 bis ≤ 25 Gew.-% kationische Gruppen enthaltender sekundärer Emulgatorkomponente (b), bezogen auf die Summe dieser Komponenten.

5. Eine Öl-in-Wasser-Emulsion nach mindestens einem der Ansprüche 1 bis 4, **dadurch gekennzeichnet, dass** die primäre nichtionische Emulgatorkomponente (a) aus einer Mischung von ≥ 25 bis ≥ 100 Gew.-% Polyglycerinpartialestern und ≥ 0 bis ≥ 75 Gew.-% Sorbitanestern besteht, die als hydrophobe Anteile aliphatische, lineare oder verzweigte, ggfs. ungesättigte und/oder hydroxyfunktionalisierte Carbonsäuren mit einer Kettenlänge von ≥ 8 bis ≥ 18 C-Atomen besteht.

6. Eine Öl-in-Wasser-Emulsion nach einem oder mehreren der Ansprüche 1 bis 5, **dadurch gekennzeichnet, dass** die primäre nichtionische Emulgatorkomponente (a) aus Polyglycerinlauraten oder Mischungen von Polyglycerinlauraten mit Sorbitanlauraten besteht.

7. Eine Öl-in-Wasser-Emulsion nach mindestens einem der Ansprüche 1 bis 6, **dadurch gekennzeichnet, dass** mindestens ein aliphatisches Cotensid (B) eingesetzt wird ausgewählt aus n-Pentanol, n-Hexanol, 1,2-Hexandiol, 1,2-Heptandiol und 1,2-Octandiol.

8. Eine Öl-in-Wasser-Emulsion nach mindestens einem der Ansprüche 1 bis 7, **dadurch gekennzeichnet, dass** mindestens ein aromatisches Cotensid (B) eingesetzt wird.

9. Eine Öl-in-Wasser-Emulsion nach mindestens einem der Ansprüche 1 bis 8, **dadurch gekennzeichnet, dass** als aromatische Cotenside (B) Phenoxyethanol, Phenoxyisopropanol, Benzylalkohol, Alkylparabenester alleine oder in Mischungen miteinander oder in Mischungen mit aliphatischen Cotensiden eingesetzt werden.

10. Eine Öl-in-Wasser-Emulsion nach mindestens einem der Ansprüche 1 bis 9, **dadurch gekennzeichnet, dass** als Öle (C) kosmetische Esteröle, Etheröle oder Mineralöle eingesetzt werden.

11. Eine Öl-in-Wasser-Emulsion nach mindestens einem der Ansprüche 1 bis 10, **dadurch gekennzeichnet, dass** als Öl mindestens eine Verbindung eingesetzt wird, ausgesucht aus der Gruppe Ethylhexylpalmitat, Ethylhexylstearat, Decylcocoat, Diethylhexylcarbonat, Dioctylcarbonat, Cetearylethylhexanoat, Decyloleat, Isocetylpalmitat, Cetearylisononanoat, Hexyllaurat, Isopropylisononaoat, Isopropylstearat, Isopropylpalmitat, Isoproylmyristat, Isopropyllaurat, C₁₂₋₁₅-Alkylbenzoat, Dicaprylyl Ether, Mineralöl, Isohexadecan, Cyclopentasiloxan, Octyldodecanol oder Mischungen dieser Verbindungen.

12. Im Wesentlichen von ethoxylierten Inhaltsstoffen freie Ölphasen enthaltend
A) eine Emulgatormischung bestehend aus
a) und b), wie in Anspruch 1 definiert,
B) ein oder mehrere Cotenside und
C) ein oder mehrere Öle und
D) ≥ 0 bis < 10 Gew.-%, bezogen auf die gesamte Ölphase, eines oder mehrerer polarer Lösungsvermittler und gegebenfalls
E) übliche Hilfs- und Zusatzstoffe.

13. Ölphasen gemäß Anspruch 12, **dadurch gekennzeichnet, dass** sie homogen und klar sind.

14. Verfahren zur Herstellung von Öl-in-Wasser-Emulsionen, **dadurch gekennzeichnet, dass** Ölphasen nach Anspruch 12 oder 13 mit einer entsprechenden Wasserphase unter an sich bekannten Bedingungen auf einen Gesamtwasserphasengehalt von ≥ 70 Gew.-% eingestellt werden.

15. Verfahren zur Herstellung klarer bis transparenter mikroemulsionsartiger Konzentrate
enthaltend
A) eine Emulgatormischung bestehend aus
a) und b), wie in Anspruch 1 definiert,
B) ein oder mehrere Cotenside und
C) ein oder mehrere Öle und gegebenfalls
D) einen oder mehrere polare Lösungsvermittler und gegebenfalls
E) übliche Hilfs- und Zusatzstoffe,
, **dadurch gekennzeichnet, dass** Wasser in eine Ölphase nach Anspruch 12 oder 13 bei Raumtemperatur eingerührt wird, mit der Maßgabe, dass der Gesamtwasserphasengehalt der mikroemulsionsartigen Konzentrate ≥ 10 bis < 70 Gew.-%, bezogen auf das gesamte Konzentrat, beträgt.

16. Verfahren zur Herstellung von Öl-in-Wasser-Emulsionen, **dadurch gekennzeichnet, dass** klare bis transparente mikroemulsionsartige Konzentrate, hergestellt nach Anspruch 15, mit einer entsprechenden Wasserphase unter an sich bekannten Bedingungen auf einen Gesamtwasserphasengehalt von ≥ 70 Gew.-% eingestellt werden.

17. Verwendung der Öl-in-Wasser-Emulsionen gemäß mindestens einem der Ansprüche 1 bis 11 zur Herstellung von kosmetischen, dermatologischen oder pharmazeutischen Zubereitungen.

18. Verwendung von Öl-in-Wasser-Emulsionen gemäß Anspruch 17 zur Herstellung von Tränktüchern oder in sprühbaren Zubereitungen für Gesichts- und Körperpflege, Babypflege, Sonnenschutz, Make-Up Remover, Antiperspirantien/Deodorantien.

19. Verwendung von Öl-in-Wasser-Emulsionen nach einem der Ansprüche 1 bis 11 zur Herstellung von Reinigungs- und Pflegemitteln für Haushalt und Industrie.

20. Verwendung von Öl-in-Wasser Emulsionen gemäß Anspruch 19 zur Herstellung von Tränktüchern oder in sprühbaren Zubereitungen zur Reinigung und Pflege von Textilien, Leder, Kunststoffen, metallischen und nichtmetallischen Oberflächen.

## Claims

1. Prolonged-stability, low-viscosity, fine oil-in-water emulsions which are essentially free of ethoxylated ingredients and have a mean radius of the emulsion droplets of from ≥ 20 to ≤ 500 nm, comprising
A) an emulsifier mixture consisting of
a) at least one nonionic primary emulsifier selected from the following groups
a1) glyceryl and polyglyceryl partial esters, preferably prepared by esterifying aliphatic, linear or branched, optionally unsaturated and/or hydroxy-functionalized carboxylic acids having a chain length of from ≥ 6 to ≤ 22 carbon atoms with glycerol, polyglycerols or mixtures of the two,
a2) sorbitan or sorbitol partial esters, preferably prepared by esterifying aliphatic, linear or branched, optionally unsaturated and/or hydroxy-functionalized carboxylic acids having a chain length of from ≥ 6 to ≤ 22 carbon atoms with sorbitol,
a3) carbohydrate esters, preferably glycoside or sucrose esters, preferably prepared by esterifying aliphatic, linear or branched, optionally unsaturated and/or hydroxy-functionalized carboxylic acids having a chain length of from ≥ 6 to ≤ 22 carbon atoms with mono-, oligo- or polysaccharides,
a4) (alkylpoly)glycosides, preferably prepared by reacting aliphatic, linear or branched, optionally unsaturated and/or additionally hydroxy-functionalized alcohols having a chain length of from ≥ 6 to ≤ 22 carbon atoms with mono- or polysaccharides, or mixtures thereof, and
b) at least one secondary emulsifier containing cationic groups, comprising one or more emulsifiers which are selected from at least one of the groups of
b1) quaternary ammonium compounds of the general formula I 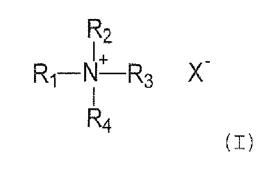 in which
R₁is selected from the group of
- branched or unbranched, cyclic or acyclic C₆₋₂₂-alkyl or -alkenyl radical,
- R₅-COO-R₆- where R₅ = branched or unbranched, cyclic or acyclic, optionally hydroxy-functional C₅₋₂₁-alkyl or -alkenyl radical and R₆ = branched or unbranched C₂₋₅-alkylene radical,
- R₅-CONH-R₆- where R₅ = branched or unbranched, cyclic or acyclic, optionally hydroxy-functional C₅₋₂₁-alkyl or -alkenyl radical and R₆ = branched or unbranched C₂₋₅-alkylene radical,
R₂, R₃, R₄ are each independently selected from the group of
- branched or unbranched, cyclic or acyclic C₆₋₂₂-alkyl or -alkenyl radical,
- R₅-COO-R₆- where R₅ = branched or unbranched, cyclic or acyclic, optionally hydroxy-functional C₅₋₂₁-alkyl or -alkenyl radical and R₆ = branched or unbranched C₂₋₅-alkylene radical,
- R₅-CONH-R₆- where R₅ = branched or unbranched, cyclic or acyclic, optionally hydroxy-functional C₅₋₂₁-alkyl or -alkenyl radical and R₆ = branched or unbranched C₂₋₅-alkylene radical,
- C₁₋₄-alkyl radical,
- C₂₋₄-alkylene radical,
- H,
- 2-hydroxyethyl radical or 2-hydroxypropyl radical,
X⁻ is an anion compatible with the quaternary ammonium compound, for example halide, methylsulfate, ethylsulfate, glycolate, lactate, acetate, sulfate, nitrate or phosphate,
b2) pyridinium, oxazolinium or thiazolinium compounds containing one or more alkyl radicals having from ≥ 6 to ≤ 22 carbon atoms,
b3) alkylguanidinium salts containing alkyl chains having from ≥ 6 to ≤ 22 carbon atoms,
b4) benzylammonium compounds having one or more alkyl radicals having from ≥ 6 to ≤ 22 carbon atoms,
b5) amine oxide salts containing one or more alkyl radicals having from ≥ 6 to ≤ 22 carbon atoms,
b6) silicone-based polymers containing at least one quaternary ammonium group, a protonated amino group or an alkylguanidinium group,
b7) compounds of the formula II 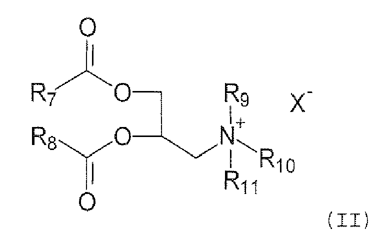 in which
R₇ and R₈ are each independently branched or unbranched, cyclic or acyclic C₆₋₂₂-alkyl or - alkenyl radicals,
R₉, R₁₀ and R₁₁ are each independently C₁₋₄-alkyl radicals,
b8) compounds of the formula III 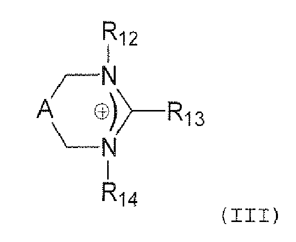 in which
A is a direct single or double bond,
R₁₂ is H or C₁₋₄-alkyl,
R₁₃ is H or C₁₋₂₂-alkyl,
R₁₄ is selected from the group of
- H,
- C₁₋₂₂-alkyl,
- (CH₂)₂-NHCO-R₁₅ where R₁₅ = branched or unbranched, cyclic or acyclic, optionally hydroxy-functional C₅₋₂₁-alkyl or -alkenyl radical,
- COR₁₅ where R₁₅ = branched or unbranched, cyclic or acyclic, optionally hydroxy-functional C₅₋₂₁-alkyl or -alkenyl radical,
with the proviso that at least one of the R₁₂, R₁₃ and R₁₄ radicals contains at least 6 carbon atoms,
b9) compounds of the formula IV 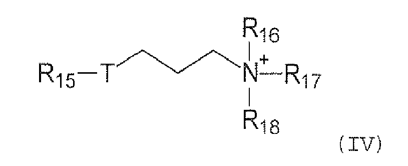 in which
R₁₅ is branched or unbranched C₆₋₂₂-alkyl or -alkenyl radical,
T is NH or O,
R₁₆, R₁₇, R₁₈ are each independently H, C₁₋₄-alkyl or 2-hydroxyalkyl,
b10) salts of compounds of the formula R₁₉-O-(CH₂)₃-NH- (CH₂)₃-NH₂ where R₁₉ = branched or unbranched C₆₋₂₂-alkyl or -alkenyl radical in which at least one of the two amine groups is present in protonated form,
b11) compounds of the general formula V 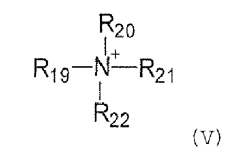 in which
R₁₉ is selected from
- branched or unbranched C₆₋₂₂-alkyl or -alkenyl radical or
- R₂₃-CONH- (CH₂)n- where R₂₃ = branched or unbranched C₅₋₂₁-alkyl or -alkenyl radical and n = 2 to 4,
R₂₀ and R₂₁ are each independently selected from H, C₁₋₄-alkyl radical, C₂₋₄-alkenyl radical or C₂₋₃-hydroxyalkyl radical and
R₂₂ is selected from -CH₂COO⁻, -(CH₂)₂COO⁻, -(CH₂)₃SO₃⁻ and -CH₂CH₂OHCH₂SO₃⁻,
B) one or more cosurfactants and
C) one or more oils and optionally
D) one or more polar solubilizers and optionally
E) customary assistants and additives,
with the proviso that the water phase content of the emulsions is ≥ 70% by weight based on the overall emulsion.

2. Oil-in-water emulsion according to Claim 1, **characterized in that** it has a water phase content of from ≥ 80 to ≤ 99% by weight.

3. Oil-in-water emulsion according to Claim 1 or 2, **characterized in that** emulsifier mixture (A), cosurfactants (B) and oils (C) are present in proportions by weight of from ≥ 10 to ≤ 30 (A), from ≥ 1 to ≤ 20 (B) and from ≥ 50 to ≤ 89 (C) based on the sum of these components.

4. Oil-in-water emulsion according to one or more of Claims 1 to 3, **characterized in that** the emulsifier mixture (A) is composed of from ≥ 75 to ≤ 99.9% by weight of nonionic primary emulsifier component (a) and from ≥ 0.1 to ≤ 25% by weight of secondary emulsifier component (b) containing cationic groups, based on the sum of these components.

5. Oil-in-water emulsion according to at least one of Claims 1 to 4, **characterized in that** the primary nonionic emulsifier component (a) consists of a mixture of from ≥ 25 to ≤ 100% by weight of polyglyceryl partial esters and from ≥ 0 to ≤ 75% by weight of sorbitan esters which contain, as hydrophobic moieties, aliphatic, linear or branched, optionally unsaturated and/or hydroxy-functionalized carboxylic acids having a chain length of from ≥ 8 to ≤ 18 carbon atoms.

6. Oil-in-water emulsion according to one or more of Claims 1 to 5, **characterized in that** the primary nonionic emulsifier component (a) consists of polyglyceryl laurates or mixtures of polyglyceryl laurates with sorbitan laurates.

7. Oil-in-water emulsion according to at least one of Claims 1 to 6, **characterized in that** at least one aliphatic cosurfactant (B) selected from n-pentanol, n-hexanol, 1,2-hexanediol, 1,2-heptanediol and 1,2-octanediol is used.

8. Oil-in-water emulsion according to at least one of Claims 1 to 7, **characterized in that** at least one aromatic cosurfactant (B) is used.

9. Oil-in-water emulsion according to at least one of Claims 1 to 8, **characterized in that** the aromatic cosurfactants (B) used are phenoxyethanol, phenoxyisopropanol, benzyl alcohol, alkylparaben esters alone or in mixtures with one another or in mixtures with aliphatic cosurfactants.

10. Oil-in-water emulsion according to at least one of Claims 1 to 9, **characterized in that** the oils (C) used are cosmetic ester oils, ether oils or mineral oils.

11. Oil-in-water emulsion according to at least one of claims 1 to 10, **characterized in that** the oil used is at least one compound selected from the group of ethylhexyl palmitate, ethylhexyl stearate, decyl cocoate, diethylhexyl carbonate, dioctyl carbonate, cetearyl ethylhexanoate, decyl oleate, isocetyl palmitate, cetearyl isononanoate, hexyl laurate, isopropyl isononanoate, isopropyl stearate, isopropyl palmitate, isopropyl myristate, isopropyl laurate, C₁₂₋₁₅ alkyl benzoate, dicaprylyl ether, mineral oil, isohexadecane, cyclopentasiloxane, octyldodecanol or mixtures of these compounds.

12. Oil phases essentially free of ethoxylated ingredients, comprising
A) an emulsifier mixture consisting of
a) and b), as defined in Claim 1,
B) one or more cosurfactants and
C) one or more oils and
D) from ≥ 0 to < 10% by weight, based on the overall oil phase, of one or more polar solubilizers and optionally
E) customary assistants and additives.

13. Oil phases according to Claim 12, **characterized in that** they are homogeneous and clear.

14. Process for preparing oil-in-water emulsions, **characterized in that** oil phases according to Claim 12 or 13 are adjusted to a total water phase content of ≥ 70% by weight with an appropriate water phase under conditions known per se.

15. Process for preparing clear to transparent microemulsion-like concentrates
comprising
A) an emulsifier mixture consisting of
a) and b), as defined in Claim 1,
B) one or more cosurfactants and
C) one or more oils and optionally
D) one or more polar solubilizers and optionally
E) customary assistants and additives,
**characterized in that** water is stirred into an oil phase according to Claim 12 or 13 at room temperature, with the proviso that the total water phase content of the microemulsion-like concentrates is from ≥ 10 to < 70% by weight, based on the overall concentrate.

16. Process for preparing oil-in-water emulsions, **characterized in that** clear to transparent microemulsion-like concentrates, prepared according to Claim 15, are adjusted to a total water phase content of ≥ 70% by weight with an appropriate water phase under conditions known per se.

17. Use of the oil-in-water emulsions according to at least one of Claims 1 to 11 for producing cosmetic, dermatological or pharmaceutical formulations.

18. Use of oil-in-water emulsions according to Claim 17 for producing impregnated wipes or in sprayable formulations for face- and bodycare, babycare, sun protection, makeup removers, antiperspirants/deodorants.

19. Use of oil-in-water emulsions according to one of Claims 1 to 11 for producing cleaning compositions and care compositions for the household and industry.

20. Use of oil-in-water emulsions according to Claim 19 for producing impregnated wipes or in sprayable formulations for the cleaning and care of textiles, leather, plastics, metallic and nonmetallic surfaces.

## Revendications

1. Émulsions huile dans eau stables à long terme, peu visqueuses, finement divisées, essentiellement exemptes de composants éthoxylés, présentant un rayon moyen des gouttes d'émulsion de ≥ 20 à ≤ 500 nm, contenant
A) un mélange d'émulsifiants constitué par
a) au moins un émulsifiant primaire non ionique choisi dans les groupes suivants
a1) les esters partiels de glycérine et de polyglycérine, de préférence fabriqués par estérification d'acides carboxyliques aliphatiques, linéaires ou ramifiés, éventuellement insaturés et/ou à fonction hydroxy, d'une longueur de chaîne de ≥ 6 à ≤ 22 atomes C, avec de la glycérine, des polyglycérines ou des mélanges des deux,
a2) le sorbitane ou les esters partiels de sorbitol, de préférence fabriqués par estérification d'acides carboxyliques aliphatiques, linéaires ou ramifiés, éventuellement insaturés et/ou à fonction hydroxy, d'une longueur de chaîne de ≥ 6 à ≤ 22 atomes C, avec du sorbitol,
a3) les esters d'hydrates de carbone, de préférence les esters de glycoside ou de sucrose, de préférence fabriqués par estérification d'acides carboxyliques aliphatiques, linéaires ou ramifiés, éventuellement insaturés et/ou à fonction hydroxy, d'une longueur de chaîne de ≥ 6 à ≤ 22 atomes C, avec des mono-, oligo-ou polysaccharides,
a4) les (alkylpoly)-glycosides, de préférence fabriqués par mise en réaction d'alcools aliphatiques, linéaires ou ramifiés, éventuellement insaturés et/ou à fonction hydroxy supplémentaire, d'une longueur de chaîne de ≥ 6 à ≤ 22 atomes C, avec des mono- ou polysaccharides,
ou leurs mélanges, et
b) au moins un émulsifiant secondaire contenant des groupes cationiques, contenant un ou plusieurs émulsifiants choisis dans au moins un des groupes
b1) les composés d'ammonium quaternaires de formule générale I 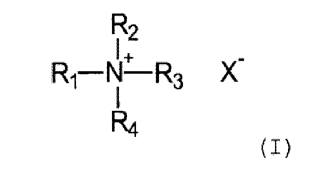 dans laquelle
R₁ est choisi dans le groupe constitué par
- radical alkyle ou alcényle en C₆₋₂₂ éventuellement ramifié, éventuellement cyclique,
- R₅-COO-R₆- avec R₅ = radical alkyle ou alcényle en C₅₋₂₁ éventuellement ramifié, éventuellement cyclique, éventuellement à fonction hydroxy, et R₆ = radical alkylène en C₂₋₅ éventuellement ramifié,
- R₅-CONH-R₆- avec R₅ = radical alkyle ou alcényle en C₅₋₂₁ éventuellement ramifié, éventuellement cyclique, éventuellement à fonction hydroxy, et R₆ = radical alkylène en C₂₋₅ éventuellement ramifié,
R₂, R₃, R₄ sont choisis indépendamment les uns des autres dans le groupe constitué par
- radical alkyle ou alcényle en C₆₋₂₂ éventuellement ramifié, éventuellement cyclique,
- R₅-COO-R₆- avec R₅ = radical alkyle ou alcényle en C₅₋₂₁ éventuellement ramifié, éventuellement cyclique, éventuellement à fonction hydroxy, et R₆ = radical alkylène en C₂₋₅ éventuellement ramifié,
- R₅-CONH-R₆- avec R₅ = radical alkyle ou alcényle en C₅₋₂₁ éventuellement ramifié, éventuellement cyclique, éventuellement à fonction hydroxy, et R₆ = radical alkylène en C₂₋₅ éventuellement ramifié,
- radical alkyle en C₁₋₄,
- radical alcényle en C₂₋₄,
- H,
- radical 2-hydroxyéthyle ou radical 2-hydroxypropyle,
X⁻ représente un anion compatible avec le composé d'ammonium quaternaire, tel que p. ex. halogénure, méthylsulfate, éthylsulfate, glycolate, lactate, acétate, sulfate, nitrate ou phosphate,
b2) les composés de pyridinium, d'oxazolium ou de thiazolinium contenant un ou plusieurs radicaux alkyle de ≥ 6 à ≤ 22 atomes C,
b3) les sels d'alkylguanidinium contenant des chaînes alkyle de ≥ 6 à ≤ 22 atomes C,
b4) les composés de benzylammonium contenant un ou plusieurs radicaux alkyle de ≥ 6 à ≤ 22 atomes C,
b5) les sels d'aminoxyde contenant un ou plusieurs radicaux alkyle de ≥ 6 à ≤ 22 atomes C,
b6) les polymères à base de silicone, contenant au moins un groupe ammonium quaternaire, un groupe amino protoné ou un groupe alkylguanidinium,
b7) les composés de formule II 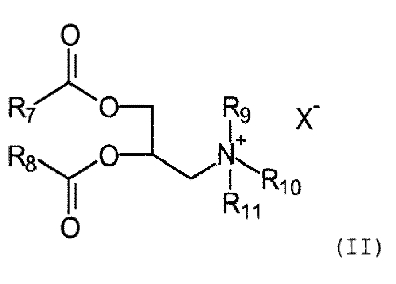 dans laquelle
R₇ et R₈ représentent indépendamment l'un de l'autre des radicaux alkyle ou alcényle en C₆₋₂₂ éventuellement ramifiés, éventuellement cycliques,
R₉, R₁₀ et R₁₁ représentent indépendamment les uns des autres des radicaux alkyle en C₁₋₄,
b8) les composés de formule III 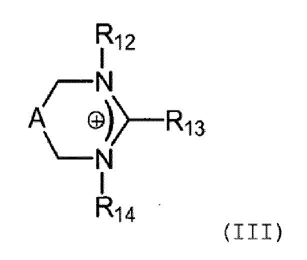 dans laquelle
A représente une simple ou double liaison directe,
R₁₂ représente H ou alkyle en C₁₋₄,
R₁₃ représente H ou alkyle en C₁₋₂₂,
R₁₄ est choisi dans le groupe constitué par
- H,
- alkyle en C₁₋₂₂,
- (CH₂)₂-NHCO-R₁₅ avec R₁₅ = radical alkyle ou alcényle en C₅₋₂₁ éventuellement ramifié, éventuellement cyclique, éventuellement à fonction hydroxy,
- COR₁₅ avec R₁₅ = radical alkyle ou alcényle en C₅₋₂₁ éventuellement ramifié, éventuellement cyclique, éventuellement à fonction hydroxy,
à condition qu'au moins un des radicaux R₁₂, R₁₃ et R₁₄ contienne au moins 6 atomes C,
b9) les composés de formule IV 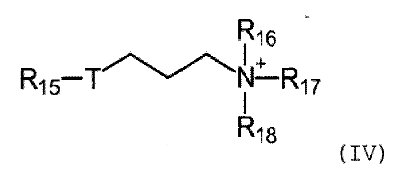 dans laquelle
R₁₅ représente un radical alkyle ou alcényle en C₆₋₂₂ éventuellement ramifié,
T représente NH ou O,
R₁₆, R₁₇, R₁₈ représentent indépendamment les uns des autres H, alkyle en C₁₋₄ ou 2-hydroxyalkyle,
b10) les sels de composés de formule R₁₉-O-(CH₂)₃-NH-(CH₂)₃-NH₂ avec R₁₉ = radical alkyle ou alcényle en C₆₋₂₂ éventuellement ramifié, dans lesquels au moins un des deux groupes amino se présente sous forme protonée, b11) les composés de formule générale V 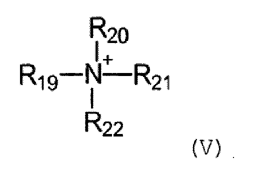 dans laquelle
R₁₉ est choisi parmi
- radical alkyle ou alcényle en C₆₋₂₂ éventuellement ramifié, ou
- R₂₃-CONH-(CH₂)ₙ- avec R₂₃ = radical alkyle ou alcényle en C₅₋₂₁ éventuellement ramifié et n = 2 à 4,
R₂₀ et R₂₁ sont choisis indépendamment l'un de l'autre parmi H, un radical alkyle en C₁₋₄, un radical alcényle en C₂₋₄ ou un radical hydroxyalkyle en C₂₋₃, et
R₂₂ est choisi parmi -CH₂COO⁻, -(CH₂)₂COO⁻, -(CH₂)₃SO₃⁻ et -CH₂CH₂OHCH₂SO₃⁻,
B) un ou plusieurs cotensioactifs, et
C) une ou plusieurs huiles, et éventuellement
D) un ou plusieurs solubilisants polaires, et éventuellement
E) des adjuvants et additifs usuels,
à condition que la teneur en phase aqueuse des émulsions soit ≥ 70 % en poids, par rapport à l'émulsion totale.

2. Émulsion huile dans eau selon la revendication 1, **caractérisée en ce qu'**elle présente une teneur en phase aqueuse de ≥ 80 à ≤ 99 % en poids.

3. Émulsion huile dans eau selon la revendication 1 ou 2, **caractérisée en ce que** le mélange d'émulsifiants (A), les cotensioactifs (B) et les huiles (C) sont présents en proportions en poids de ≥ 10 à ≤ 30 (A), ≥ 1 à ≤ 20 (B) et ≥ 50 à ≤ 89 (C), par rapport à la somme de ces composants.

4. Émulsion huile dans eau selon une ou plusieurs des revendications 1 à 3, **caractérisée en ce que** le mélange d'émulsifiants (A) est composé de ≥ 75 à ≤ 99,9 % en poids du composant émulsifiant primaire non ionique (a) et de ≥ 0,1 à ≤ 25 % en poids du composant émulsifiant secondaire contenant des groupes cationiques (b), par rapport à la somme de ces composants.

5. Émulsion huile dans eau selon au moins l'une quelconque des revendications 1 à 4, **caractérisée en ce que** le composant émulsifiant non ionique primaire (a) est constitué d'un mélange de ≥ 25 à ≤ 100 % en poids d'esters partiels de polyglycérine et de ≥ 0 à ≤ 75 % en poids d'esters de sorbitane, qui est constitué en tant que fractions hydrophobes d'acides carboxyliques aliphatiques, linéaires ou ramifiés, éventuellement insaturés et/ou à fonction hydroxy, d'une longueur de chaîne de ≥ 8 à ≤ 18 atomes C.

6. Émulsion huile dans eau selon une ou plusieurs des revendications 1 à 5, **caractérisée en ce que** le composant émulsifiant non ionique primaire (a) est constitué de laurates de polyglycérine ou de mélanges de laurates de polyglycérine avec des laurates de sorbitane.

7. Émulsion huile dans eau selon au moins l'une quelconque des revendications 1 à 6, **caractérisée en ce qu'**au moins un cotensioactif aliphatique (B) est utilisé, choisi parmi le n-pentanol, le n-hexanol, le 1,2-hexanediol, le 1,2-heptanediol et le 1,2-octanediol.

8. Émulsion huile dans eau selon au moins l'une quelconque des revendications 1 à 7, **caractérisée en ce qu'**au moins un cotensioactif aromatique (B) est utilisé.

9. Émulsion huile dans eau selon au moins l'une quelconque des revendications 1 à 8, **caractérisée en ce que** du phénoxyéthanol, du phénoxyisopropanol, de l'alcool benzylique, des esters d'alkylparabène sont utilisés seuls ou en mélange les uns avec les autres ou en mélange avec des cotensioactifs aliphatiques en tant que cotensioactifs aromatiques (B).

10. Émulsion huile dans eau selon au moins l'une quelconque des revendications 1 à 9, **caractérisée en ce que** des huiles d'esters, des huiles d'éthers ou des huiles minérales cosmétiques sont utilisées en tant qu'huiles (C).

11. Émulsion huile dans eau selon au moins l'une quelconque des revendications 1 à 10, **caractérisée en ce qu'**au moins un composé choisi dans le groupe constitué par le palmitate d'éthylhexyle, le stéarate d'éthylhexyle, le cocoate de décyle, le carbonate de diéthylhexyle, le carbonate de dioctyle, l'hexanoate de cétéaryléthyle, l'oléate de décyle, le palmitate d'isocétyle, l'isononanoate de cétéaryle, le laurate d'hexyle, l'isononanoate d'isopropyle, le stéarate d'isopropyle, le palmitate d'isopropyle, le myristate d'isopropyle, le laurate d'isopropyle, le benzoate d'alkyle en C₁₂₋₁₅, l'éther dicaprylique, une huile minérale, l'isohexadécane, le cyclopentasiloxane, l'octyldodécanol ou les mélanges de ces composés est utilisé en tant qu'huile.

12. Phases huileuses essentiellement exemptes de composants éthoxylés, contenant
A) un mélange d'émulsifiants constitué par
a) et b), tels que définis dans la revendication 1,
B) un ou plusieurs cotensioactifs et
C) une ou plusieurs huiles et
D) ≥ 0 à < 10 % en poids, par rapport à la phase huileuse totale, d'un ou de plusieurs solubilisants polaires, et éventuellement
E) des adjuvants et additifs usuels.

13. Phases huileuses selon la revendication 12, **caractérisées en ce qu'**elles sont homogènes et claires.

14. Procédé de fabrication d'émulsions huile dans eau, **caractérisé en ce que** des phases huileuses selon la revendication 12 ou 13 sont ajustées à une teneur en phase aqueuse totale ≥ 70 % en poids avec une phase aqueuse appropriée dans des conditions connues en soi.

15. Procédé de fabrication de concentrés de type microémulsion clairs à transparents, contenant
A) un mélange d'émulsifiants constitué par
a) et b), tels que définis dans la revendication 1,
B) un ou plusieurs cotensioactifs et
C) une ou plusieurs huiles, et éventuellement
D) un ou de plusieurs solubilisants polaires, et éventuellement
E) des adjuvants et additifs usuels,
**caractérisé en ce que** de l'eau est incorporée dans une phase huileuse selon la revendication 12 ou 13 à température ambiante, à condition que la teneur en phase aqueuse totale des concentrés de type microémulsion soit de ≥ 10 à < 70 % en poids, par rapport au concentré total.

16. Procédé de fabrication d'émulsions huile dans eau, **caractérisé en ce que** des concentrés de type microémulsion clairs à transparents, fabriqués selon la revendication 15, sont ajustés à une teneur en phase aqueuse totale ≥ 70 % en poids avec une phase aqueuse appropriée dans des conditions connues en soi.

17. Utilisation des émulsions huile dans eau selon au moins l'une quelconque des revendications 1 à 11 pour la fabrication de préparations cosmétiques, dermatologiques ou pharmaceutiques.

18. Utilisation d'émulsions huile dans eau selon la revendication 17, pour la fabrication de lingettes imprégnées ou dans des préparations pulvérisables pour le soin du visage et du corps, le soin de bébé, la protection solaire, les démaquillants, les anti-transpirants/déodorants.

19. Utilisation d'émulsions huile dans eau selon l'une quelconque des revendications 1 à 11 pour la fabrication d'agents de nettoyage et de soin pour la maison et l'industrie.

20. Utilisation d'émulsions huile dans eau selon la revendication 19, pour la fabrication de lingettes imprégnées ou dans des préparations pulvérisables pour le nettoyage et le soin des textiles, du cuir, des plastiques, des surfaces métalliques et non métalliques.
